# EUROPEAN PATENT SPECIFICATION

(11) **EP 4 545 122 B1**
(45) Date of publication and mention of the grant of the patent: **15.07.2026**
(21) Application number: 24223644.6
(22) Date of filing: 23.03.2022
(51) Int. Cl.: A61K 9/00, A61M 15/00

(54) **FORMULATION FOR USE IN A METHOD OF TREATING AN ANAPHYLACTIC REACTION**
FORMULIERUNG ZUR VERWENDUNG IN EINEM VERFAHREN ZUR BEHANDLUNG EINER ANAPHYLAKTISCHEN REAKTION
FORMULATION DESTINÉE À ÊTRE UTILISÉE DANS UNE MÉTHODE DE TRAITEMENT D' UNE RÉACTION ANAPHYLACTIQUE

(30) Priority: 23.03.2021 US 202163165102 P
(43) Date of publication of application: 30.04.2025
(62) Divisional of application: 22716648.5
(73) Proprietor: KOKUA PHARMA INC., Richmond, British Columbia V7C 5L9 (CA)
(72) Inventor: LUCIUK, George Harry, Richmond, British Columbia, V7C 5L9 (CA); MARTIN, Andrew Robert, Alberta, Edmonton, T6G 1H9 (CA); RUZYCKI, Conor Aidan, Alberta, Edmonton, T6G 1H9 (CA); FINLAY, Warren Hugh, Alberta, Edmonton, T6G 1H9 (CA)
(74) Representative: J A Kemp LLP

(56) References cited:
- WO-A2-2009/095681
- US-B1- 8 367 734

## Description

### 1. BACKGROUND

Anaphylaxis is a severe systemic hypersensitivity reaction that can be potentially life-threatening. Epinephrine, also known as adrenaline, is the recommended first treatment option for anaphylaxis. Treatment of anaphylaxis has been focused on early administration of epinephrine aimed at rapid attainment of peak plasma and tissue epinephrine concentrations. Currently, the first-line treatment of choice for anaphylaxis is epinephrine administered via intramuscular injection using an EpiPen^{™} or similar auto-injector device. There are, however, numerous limitations to the use of auto-injectors for self-administration of epinephrine to treat allergic emergencies.

A primary limitation is failure in patient compliance. Many individuals who carry auto-injectors fail to use them promptly in the case of an emergency, for example due to inadequate training or pain avoidance or cost. All of these factors result in delay in administering treatment and this delay is an important aspect of failing to treat anaphylaxis successfully. This is particularly true in the case of children. Moreover, many patients at risk of severe allergic reactions do not routinely carry auto-injectors, for example due to cost, lack of supply, device size, or complacency. Failure to administer epinephrine promptly for any reason in response to a severe allergic reaction poses a significant risk of morbidity or mortality.

Various devices and formulations have been devised for the delivery of epinephrine by inhalation to treat asthma. In 1956, Riker Laboratories, Inc. introduced the first pressurized metered dose inhaler (MDI) containing epinephrine, sold under the trademark MEDIHALER-EPI^{™}. Other similar over-the-counter epinephrine inhalers later came to market, including Primatene Mist^{™} and Bronkaid Mist^{™}. Historically, such inhalers typically employed chlorofluorcarbons (CFCs) as propellants to rapidly deliver aerosolized epinephrine to treat acute attacks of asthma. However, the phase-out of CFCs pursuant to the Montreal Protocol resulted in the phase-out of CFC-containing epinephrine MDIs in late 2011.

Recently Primatene Mist^{™} has been reintroduced as an over-the-counter MDI for treatment of bronchial asthma. The new version of Primatene Mist^{™} employs hydrofluoroalkane (HFA) as a more environmentally friendly propellant rather than CFCs and is described in U.S. Patent No. 8,367,734. Propellant-free inhalable formulations containing epinephrine or epinephrine salt particles, preferably in a dry powder form, are described in U.S. Patent No. 8747813. WO2009/095681 describes aerosol devices for delivering a pharmaceutically active agent.

Certain epinephrine MDI devices designed for treatment of asthma have sometimes been employed or recommended as an alternative to intramuscular injection in order to achieve circulating levels of epinephrine for treatment of anaphylaxis. However, the therapeutic effects of previously known inhalable epinephrine systems are regarded as less reliable than those of injected epinephrine for treatment of anaphylaxis, which is a life-threatening condition. Since inhalable epinephrine systems have not been shown to be clinically equivalent or superior to injectable epinephrine, they have not been adopted as a safe and reliable treatment for anaphylaxis.

There is a need in the art for epinephrine MDIs that are suitable for safely and effectively treating anaphylaxis and for methods of using the same for treatment of anaphylaxis. There is also a need in the art for improved MDIs that provide more rapid, efficient and reliable delivery of an active pharmaceutical ingredient (API), e.g., epinephrine, to therapeutic target areas such as the lung and laryngopharynx.

### 2. SUMMARY

The present disclosure describes a metered dose inhaler (MDI) comprising an API (e.g., epinephrine) formulation and methods and uses thereof. The disclosed MDI provides delivery of API (e.g., epinephrine) to the larynx and pharynx (collectively, the "laryngopharynx") and to the lungs in a more rapid, efficient and reliable manner than prior API (e.g., epinephrine) MDI devices. In one aspect, the proportion of API (e.g., epinephrine) delivered to the oral cavity is reduced. In another aspect, the proportion of API (e.g. epinephrine) delivered to the laryngopharyx and lungs is increased. In a further aspect, the proportion of API (e.g. epinephrine) delivered to the laryngopharyx and lungs is relatively independent of whether the metered dose inhaler is used while in a transverse or coaxial orientation. In yet a further aspect, the systemic delivery of API (e.g., epinephrine) occurs more rapidly and consistently than is the case with alternative modes of administration, such as intramuscular injection, e.g., via an autoinjector. Use of the disclosed MDI thus increases the speed, efficacy and reliability and reduces the number and frequency of administration doses (metered volumes of inhalation plumes) of inhaled API (e.g., epinephrine) needed for treatment of a disease, disorder, or condition that can treated by inhalation of an API (e.g., anaphylaxis). In certain embodiments, oral inhaled delivery of an API (e.g., epinephrine) via the disclosed MDI provides resolution of symptoms of an acute allergic reaction (e.g., anaphylaxis) within 5 minutes, preferably within 4 minutes, more preferably within 3 minutes, yet more preferably within 2 minutes of initiating therapy. In one embodiment the API is a compound other than epinephrine or a salt thereof that provides medicinal benefit upon administration to a subject; preferably the compound is present as a suspension. In another embodiment the API is an immunogen (e.g., a vaccine).

In one embodiment, the present disclosure provides a MDI comprising:
(a) a formulation comprising API (e.g., epinephrine or a pharmaceutically acceptable salt thereof) (i.e., an "API formulation"), such as a suspension of API (e.g., epinephrine or a pharmaceutically acceptable salt thereof); and
(b) an actuator having one or more orifices with an effective diameter of from about 0.12 mm to about 0.33 mm. In a preferred embodiment, the MDI comprises:
   (a) a canister containing a formulation comprising API (e.g., epinephrine or a pharmaceutically acceptable salt thereof); and
   (b) an actuator having one or more orifices with an effective diameter of from about 0.12 mm to about 0.33 mm. Preferably, the MDI is a pressurized metered dose inhaler.

In some aspects, the effective diameter of the one or more orifices of the actuator is from about 0.14 mm to about 0.31 mm; from about 0.16 mm to about 0.29 mm; from about 0.18 mm to about 0.22 mm; from about 0.20 mm to about 0.25 mm; or from about 0.21 mm to about 0.23 mm. In other aspects, the effective diameter of the one or more orifices of the actuator is about 0.22 mm or is 0.22 mm. In one embodiment, the one or more orifices are circular in form; alternately, the one or more orifices may be peanut-, clover-, cross- or slot-shaped. In one embodiment, the actuator has a single orifice. In another embodiment, the actuator has a plurality of orifices, e.g., 2, 3, 4, 5, 6, 7, 8, 9, or 10 orifices, which may have the same shape or a combination of different shapes, such as those specified above. In one embodiment, the actuator has a plurality of orifices (e.g., circular orifices) that are disposed linearly (e.g., aligned vertically when the MDI is used). In one embodiment, the actuator has an effective orifice diameter as aforesaid and a sump volume of 15-20 mm³, preferably 17-19 mm³, most preferably 18 mm³ and/or a land length (i.e., channel length of the outlet orifice) of 0.55-0.75 mm, preferably 0.60-0.70 mm, most preferably 0.65. In specific embodiments, the actuator has the configuration of a Bespak BK632 or BK679 actuator (comprising a single orifice of diameter 0.33 mm, a sump volume of 18 mm³ and a land length of 0.65 mm), preferably the configuration of a Bespak BK634 or BK665 actuator (comprising a single orifice of diameter 0.30 mm, a sump volume of 18 mm³ and a land length of 0.65 mm), more preferably the configuration of a Bespak BK638 (comprising a single orifice of diameter 0.25 mm, a sump volume of 18 mm³ and a land length of 0.65 mm), most preferably the configuration of a Bespak BK633 or BK671 actuator (comprising a single orifice of diameter 0.22 mm, a sump volume of 18 mm³ and a land length of 0.65 mm). In some embodiments, the actuator has a mouthpiece that extends about 1.5 to 3.5 cm, preferably about 2.0 to 3.0 cm, more preferably about 2.5 cm from the portion of the orifice that is nearest to the user.

In other aspects, the API (e.g., epinephrine) formulation in the metered dose inhaler of the present disclosure comprises:
(a) a suspension of API (e.g., epinephrine or a pharmaceutically acceptable salt thereof);
(b) a liquefied propellant; and
(c) a co-solvent.

In some aspects, the co-solvent is present from about 0.1% to about 4%; from about 0.1% to about 3%; or from about 0.1% to about 2% w/w based on the total weight of the formulation. In a preferred embodiment, the co-solvent is present at about 1% w/w based on the total weight of the formulation. In other aspects, the co-solvent is ethanol, isopropanol, propylene glycol, ethylene glycol, propane, butane, isobutene, pentane, dimethyl ether, diethyl ether, or mixtures thereof. In a preferred embodiment, the co-solvent is ethanol (preferably dehydrated ethanol).

In some aspects, the liquefied propellant is present from about 95% to about 99.5%; from about 96% to about 99%; or from about 97% to about 99% w/w based on the total weight of the formulation. In other aspects, the liquefied propellant is 1,1,1,2-tetrafluoroethane (HFA-134A), 1,1,1,2,3,3,3-heptafluoropropane (HFA-227), or a mixture thereof. In a preferred embodiment, the liquefied propellant is 1,1,1,2-tetrafluoroethane (HFA-134a).

In some aspects, the suspension of API (e.g., epinephrine or a pharmaceutically acceptable salt thereof) is present from about 0.1% to about 0.5%; from about 0.1% to about 0.4%; or from about 0.1% to about 0.3% w/w based on the total weight of the formulation. In a preferred embodiment, the API (e.g., epinephrine or pharmaceutical salt thereof) in the suspension. In some embodiments the API is epinephrine free base. In a preferred embodiment, the epinephrine or a pharmaceutically acceptable salt thereof comprises epinephrine free base at a concentration of 0.19% w/w based on the total weight of the formulation.

In further aspects, the API (e.g., epinephrine) formulation of the present disclosure may additionally comprise a surfactant, such as a surfactant selected from mono- or poly-sorbitan oleates, oleic acid, and lecithin. In a preferred embodiment, the formulation comprises polysorbate 80. In further aspects, the API (e.g., epinephrine) formulation of the present disclosure may additionally comprise an antioxidant, such as an antioxidant selected from thymol, tocopherol, ascorbyl palmitate, butylated hydroxyanisole, butylated hydroxytoluene, propyl gallate, citric acid, sodium metabisulfite and sodium sulfite. In a preferred embodiment, the formulation comprises thymol. Preferably, the formulation is free of metabisulfite; more preferably the formulation is free of sulfites.

In another embodiment, the present disclosure provides a method of administering API (e.g., epinephrine) to a patient in need thereof or a method of treating a patient in need of API (e.g., epinephrine) using the disclosed metered dose inhaler. In some aspects, the present disclosure provides a use of API (e.g., epinephrine) for treating a patient in need of API (e.g., epinephrine) using the disclosed metered dose inhaler.

In another embodiment, the present disclosure provides a method of treating a disease, disorder, or condition that can treated by inhalation of an API (e.g., an allergic reaction such as anaphylaxis) in a patient in need thereof, comprising administering a therapeutically effective amount of API (e.g., epinephrine) by oral inhalation using the disclosed metered dose inhaler. In some aspects, the present disclosure provides a use of API (e.g., epinephrine) for treating an allergic reaction (e.g., anaphylaxis) in a patient in need thereof by oral inhalation using the disclosed metered dose inhaler. In some aspects, the method of treating the allergic reaction (e.g., anaphylaxis) comprising treating or reducing the likelihood of upper airway obstruction. In other aspects, the metered dose inhaler is an orientation independent metered dose inhaler in that the amount of API (e.g., epinephrine) delivered by the metered dose inhaler to the laryngopharynx and lungs of the patient is relatively independent (i.e., varies by less than 10%) of the coaxial or transverse orientation of the metered dose inhaler; preferably, the proportion of the delivered dose of API (e.g., epinephrine) that reaches the laryngopharynx and lungs of the patient when the metered dose inhaler is in a transverse orientation is at least 95% of the proportion of the delivered dose that reaches the laryngopharynx and lungs of the patient when the metered dose inhaler is in a coaxial orientation.

In another embodiment, a patient with an allergic reaction (e.g., anaphylaxis) is treated by administering oral inhaled inhalation via the disclosed metered dose inhaler by giving 250 mg epinephrine (e.g., two puffs) periodically (e.g., every 30-90 seconds, preferably every 45-75 seconds, more preferably about every 60 seconds) until the patient experiences subjective awareness of a physiological response to treatment (e.g., awareness of increased heart rate, awareness of body or hand tremor, or awareness of both increased heart rate and body or hand tremor).

These and other aspects of this disclosure will be apparent upon reference to the following detailed description. To this end, various references are set forth herein which describe in more detail certain background information and procedures.

### 3. BRIEF DESCRIPTION OF THE FIGURES

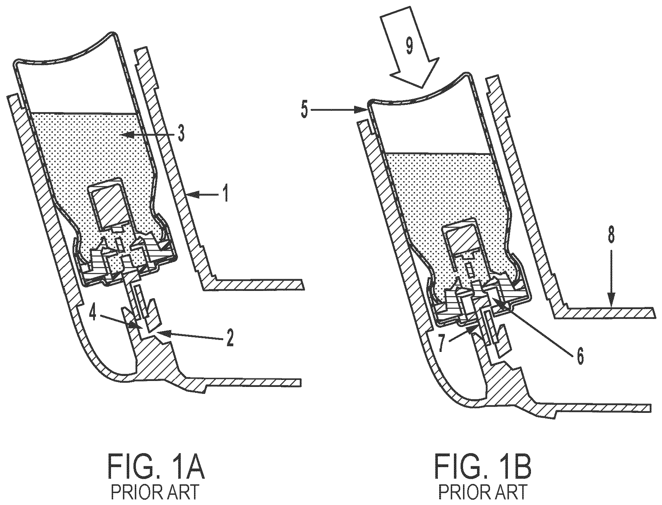

FIG. 1 provides a cross-sectional diagram of a pressured metered dose inhaler (pMDI) in resting position (panel A) and in actuation position (panel B). The MDI in this embodiment comprises actuator 1, actuator orifice 2, formulation 3, sump 4, canister 5, metered chamber 6, stem 7, and actuator mouthpiece 8. Arrow 9 shows the direction of canister 5 movement during device actuation, resulting in discharge of formulation 3 from metered chamber 6 through actuator orifice 2.

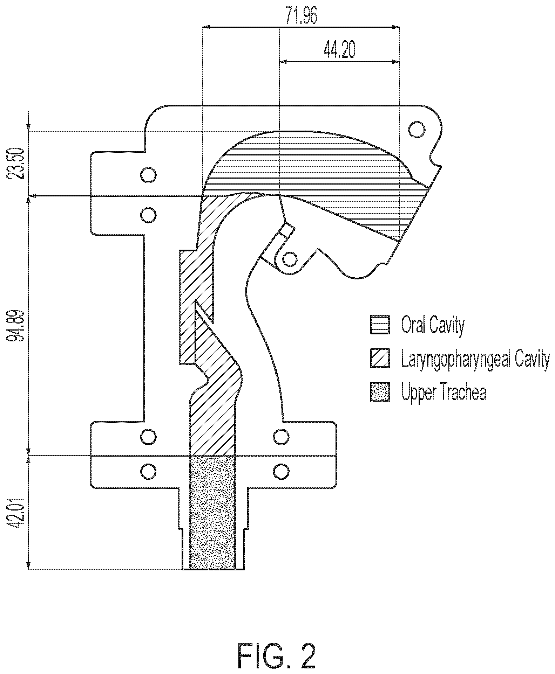

FIG. 2 provides a schematic of the Sectioned Alberta Idealized Throat (S-AIT) device showing anatomical regions. The top of the S-AIT models delivery to the oral cavity, the middle models delivery to the laryngopharynx, and the bottom models delivery to the upper trachea.

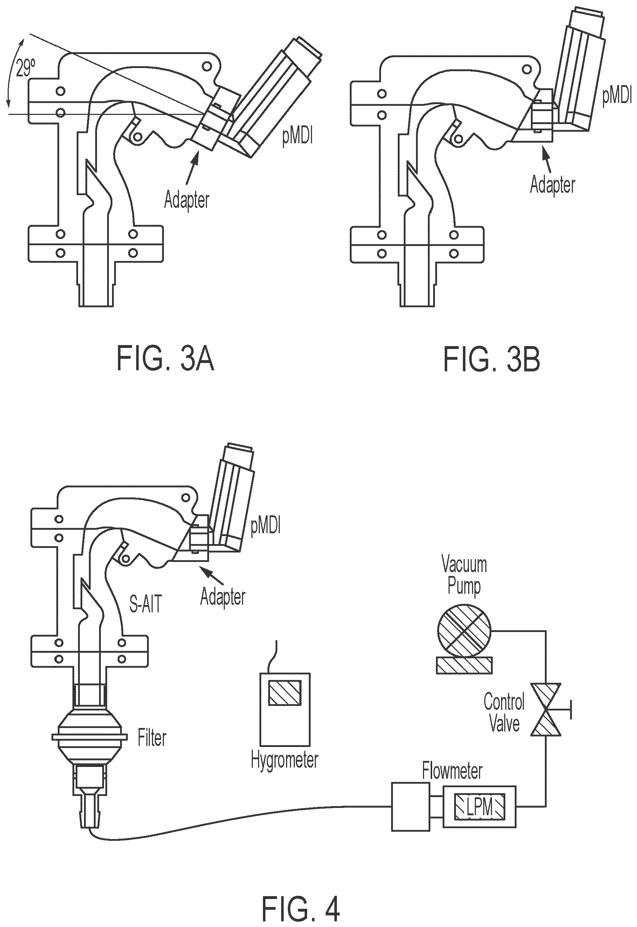

FIG. 3 provides schematics of a pressured metered dose inhaler (pMDI) applied to the S-AIT in various orientations: (a) coaxial, directed towards the back of the oral cavity, (b) transverse, oriented horizontally.
FIG. 4 provides the experimental setup used to investigate deposition of epinephrine as delivered by a pMDI using the S-AIT and filter, in which delivery to the filter models delivery to the lungs.

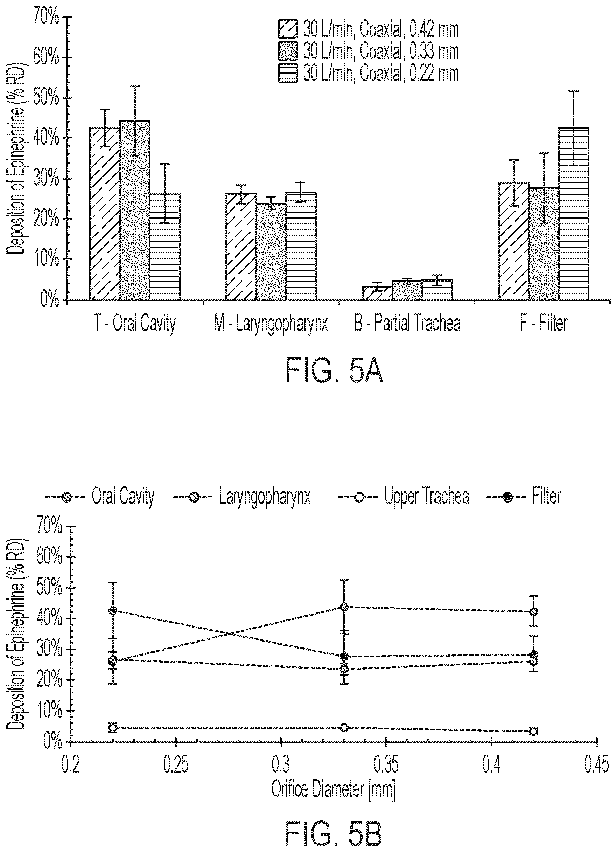

FIG. 5 provides the deposition of epinephrine in regions of interest for different actuator orifice diameters, with a coaxial insertion angle at an inhalation flowrate of 30 L/min. (a) shows a comparison of deposition recovered from each region and (b) shows the functional relationship between actuator orifice diameter and deposition in each region of interest. Error bars denote standard deviation (n = 5). RD = recovered dose.

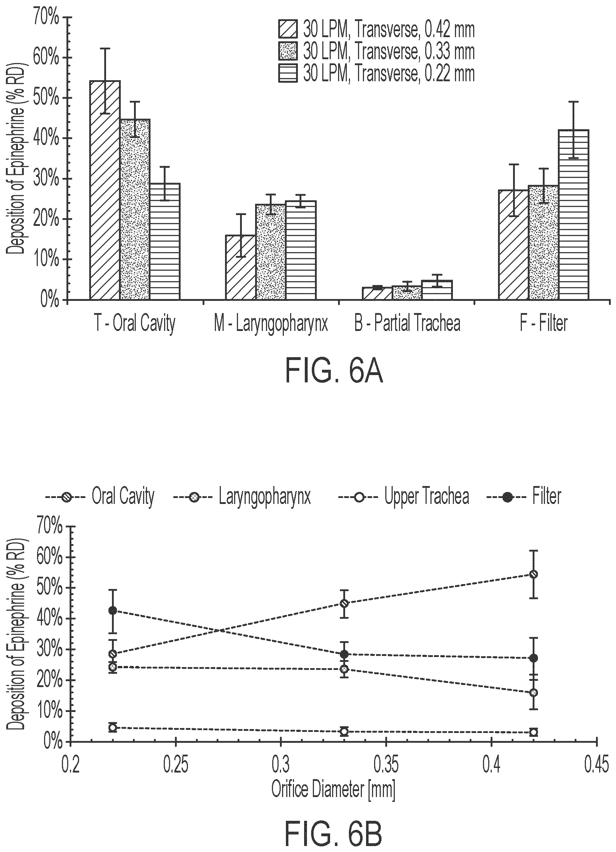

FIG. 6 provides the deposition of epinephrine in regions of interest for different actuator orifice diameters, with a transverse insertion angle at an inhalation flowrate of 30 L/min. (a) shows a comparison of deposition recovered from each region and (b) shows the functional relationship between actuator orifice diameter and deposition in each region of interest. Error bars denote standard deviation (n = 5). RD = recovered dose.

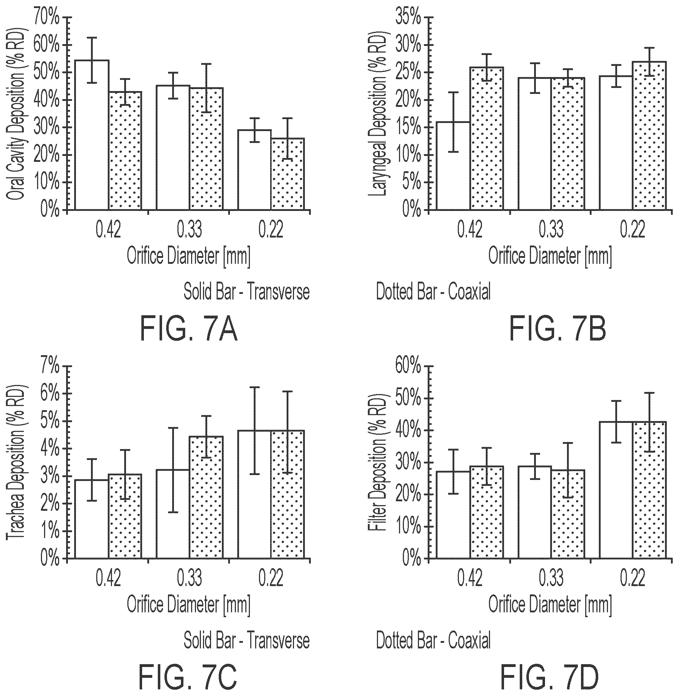

FIG. 7 provides comparisons of depositions obtained with coaxial and transverse insertion angles for each orifice diameter in (a) the oral cavity, (b) the laryngopharynx, (c) the upper trachea, and (d) the filter, expressed as a percent of the recovered dose. Error bars denote standard deviation (n = 5). RD = recovered dose.

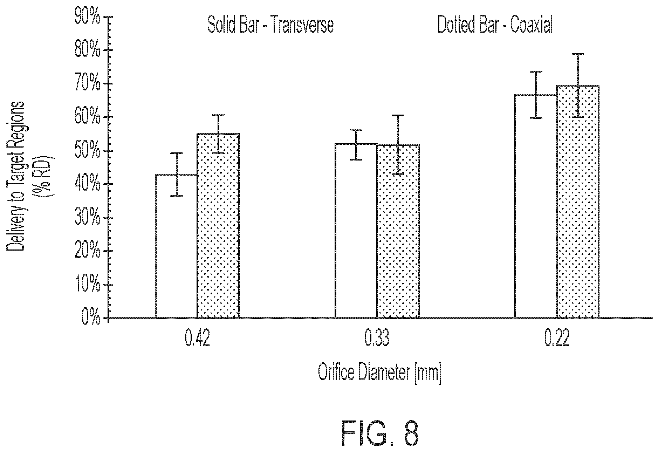

FIG. 8 provides total delivery to target regions (taken as the sum of deposition in the laryngopharynx and the filter) obtained with coaxial and transverse insertion angles for each orifice diameter at an inhalation flowrate of 30 L/min, expressed as a percent of the recovered dose. Error bars denote standard deviation. RD = recovered dose.

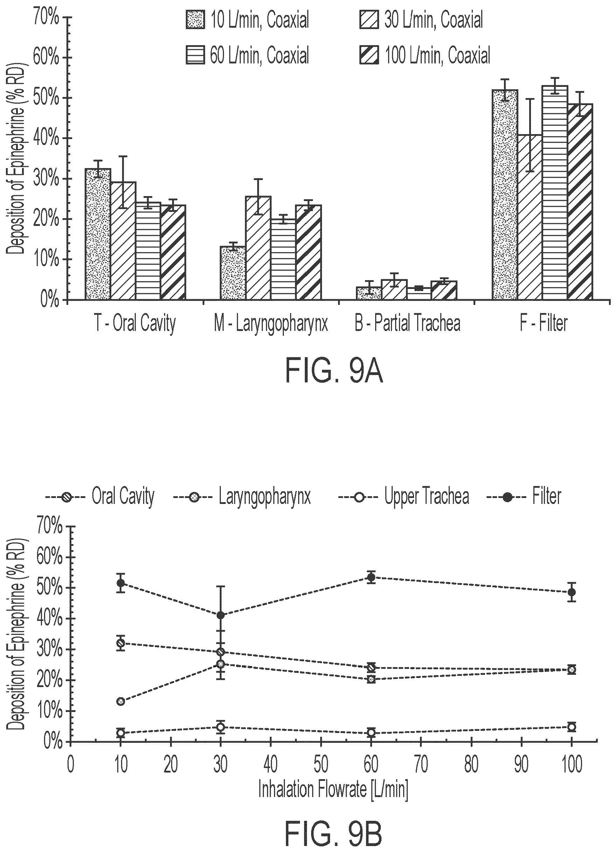

FIG. 9 provides the deposition of epinephrine in regions of interest across flowrates of interest with a coaxial insertion angle and an actuator orifice of 0.22 mm. (a) shows a comparison of deposition recovered from each region and (b) shows the functional relationship between flowrate and deposition in each region of interest. Error bars denote standard deviation (n = 5). RD = recovered dose.

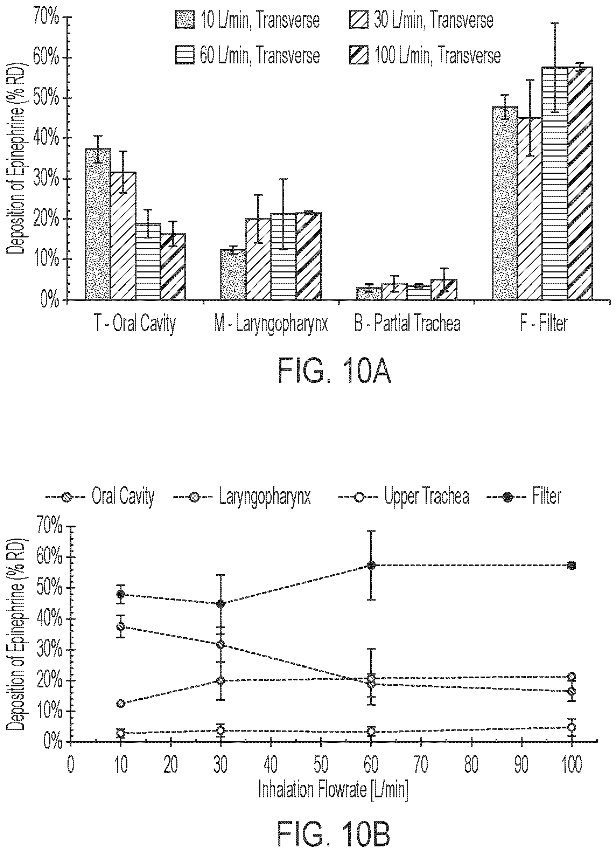

FIG. 10 provides the deposition of epinephrine in regions of interest across flowrates of interest with a transverse insertion angle and an actuator orifice of 0.22 mm. (a) shows a comparison of deposition recovered from each region and (b) shows the functional relationship between flowrate and deposition in each region of interest. Error bars denote standard deviation (n = 5). RD = recovered dose.

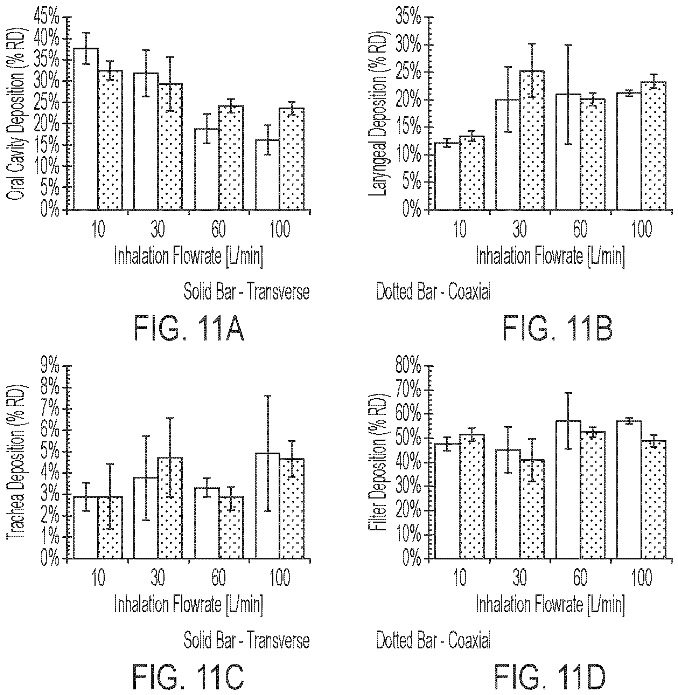

FIG. 11 provides comparisons of deposition obtained with coaxial and transverse insertion angles for the 0.22 mm actuator orifice at flowrates of interest in (a) the oral cavity, (b) the laryngopharynx, (c) the upper trachea, and (d) the filter, expressed as a percent of the recovered dose. Error bars denote standard deviation (n = 5). RD = recovered dose.

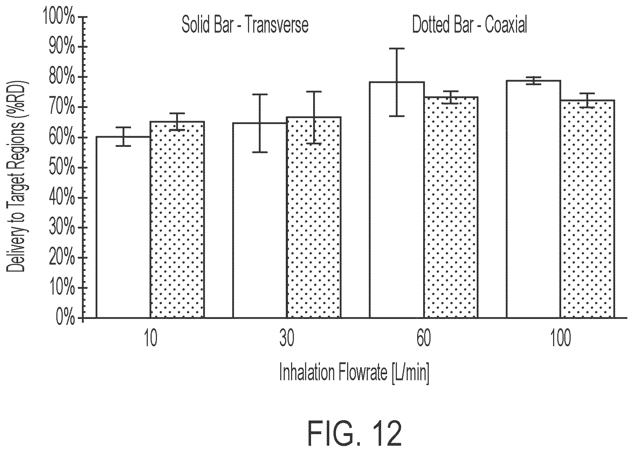

FIG. 12 provides total delivery to target regions (taken as the sum of deposition in the laryngopharynx and the filter) across flowrates of interest obtained with coaxial and transverse insertion angles for the 0.22 mm diameter orifice, expressed as a percent of the recovered dose. Error bars denote standard deviation. RD = recovered dose.

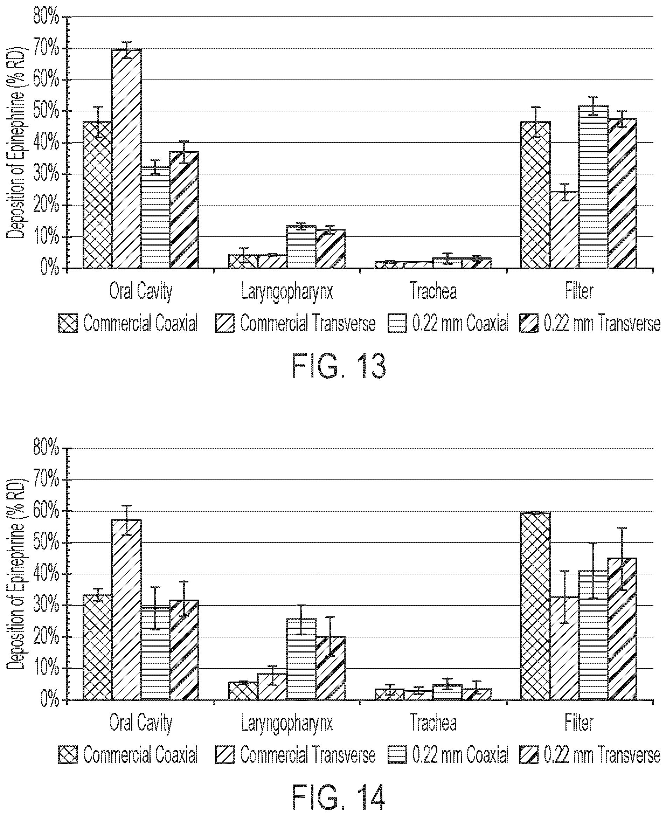

FIG. 13 provides a comparison of deposition obtained with the commercial Primatene Mist HFA actuator and the 0.22 mm orifice diameter actuator in regions of interest at 10 L/min. RD = recovered dose. Error bars denote standard deviation. RD = recovered dose.
FIG. 14 provides a comparison of deposition obtained with the commercial Primatene Mist HFA actuator and the 0.22 mm orifice diameter actuator in regions of interest at 30 L/min. RD = recovered dose. Error bars denote standard deviation. RD = recovered dose.

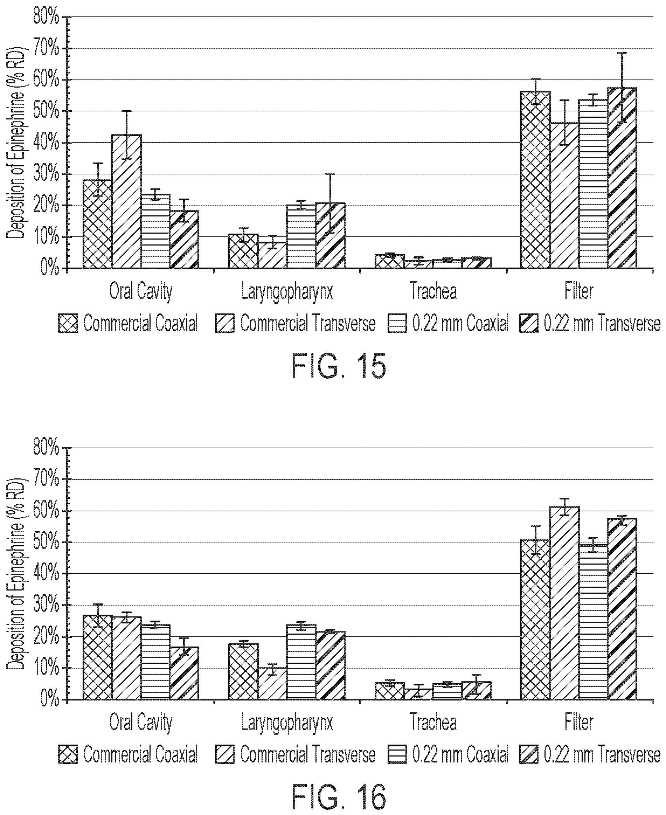

FIG. 15 provides a comparison of deposition obtained with the commercial Primatene Mist HFA actuator and the 0.22 mm orifice diameter actuator in regions of interest at 60 L/min. RD = recovered dose. Error bars denote standard deviation. RD = recovered dose.
FIG. 16 provides a comparison of deposition obtained with the commercial Primatene Mist HFA actuator and the 0.22 mm orifice diameter actuator in regions of interest at 100 L/min. RD = recovered dose. Error bars denote standard deviation. RD = recovered dose.

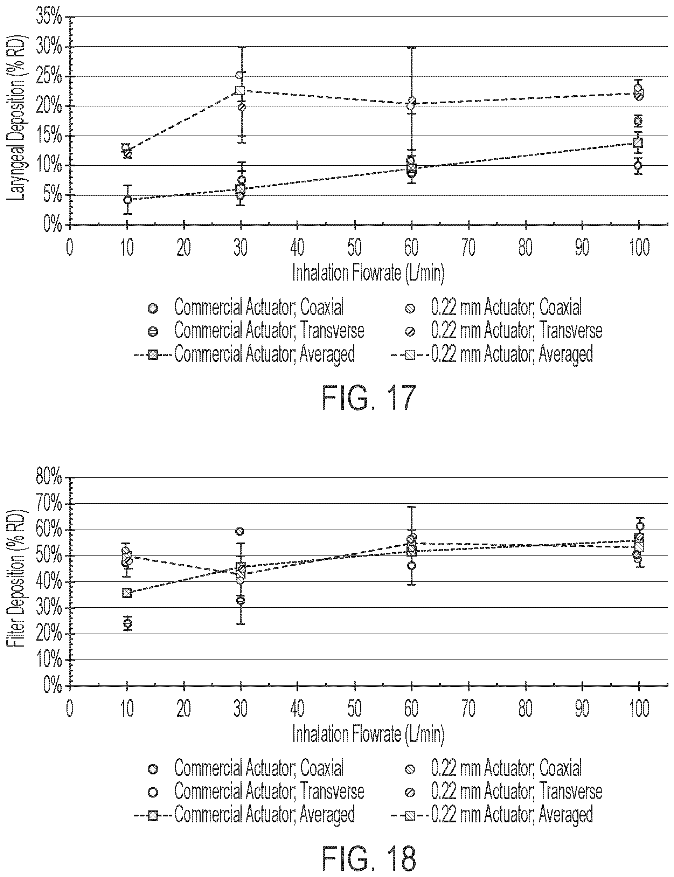

FIG. 17 provides the laryngopharyngeal deposition obtained with the commercial Primatene Mist HFA actuator versus the 0.22 mm orifice diameter actuator across all examined flowrates. Data at both the coaxial and transverse orientation angles is presented, together with the average value across both orientations. Error bars denote standard deviation. RD = recovered dose.
FIG. 18 provides the filter deposition obtained with the commercial Primatene Mist HFA actuator versus the 0.22 mm orifice diameter actuator across all examined flowrates. Data at both the coaxial and transverse orientation angles is presented, together with the average value across both orientations. Error bars denote standard deviation. RD = recovered dose.

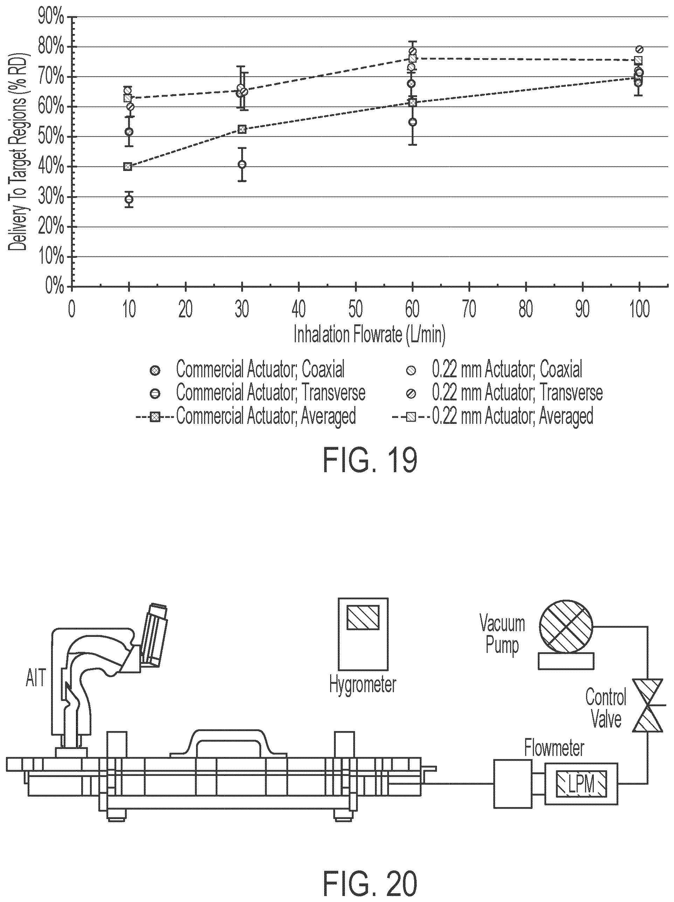

FIG. 19 provides the combined laryngopharyngeal and filter deposition obtained with the commercial Primatene Mist HFA actuator versus the 0.22 mm orifice diameter actuator across all examined flowrates. Data at both the coaxial and transverse orientation angles is presented, together with the average value across both orientations. Error bars denote standard deviation. RD = recovered dose.
FIG. 20 provides the experimental setup used to measure particle size distributions with the Next Generation Impactor placed downstream of a non-sectioned Adult Alberta Idealized Throat.

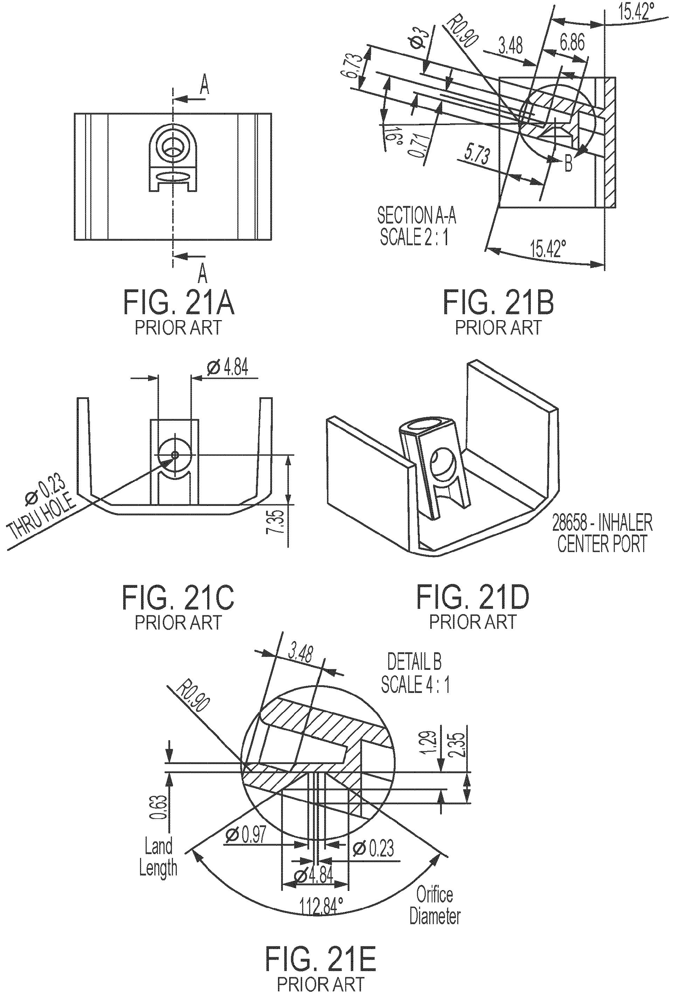

FIG. 21 depicts several views (panels A-E) of a Bespak BK633 actuator.

### 4. DETAILED DESCRIPTION

### 4.1. Definitions

As used in the specification and appended claims, unless specified to the contrary, the following terms and abbreviations have the meaning indicated:

"About" as used herein refers to a value that is within 10% of the stated number or range.

"Active pharmaceutical ingredient" ("API"), as used herein, is a chemical, biological or pharmaceutical entity including any natural or synthetic chemical or biological substance that has a pharmaceutical effect. Typical APIs include but are not limited to antibodies, antigens, biological materials, chemical materials, drugs, enzymes, hormones, immunogens, probes, tracers, nucleic acids, peptides, proteins, selective toxins and toxins. In certain embodiments, the API is epinephrine or a pharmaceutically acceptable salt thereof. In other embodiment, the API is a compound other than epinephrine or a salt thereof that provides medicinal benefit upon administration to a subject; preferably the compound is present as a suspension. In a further embodiment the API is an immunogen (e.g., a vaccine).

"API formulation" as used herein refers to a formulation comprising an API.

"Effective diameter" as used herein, refers to the diameter of a circle having an area equal to the area of the orifice (where the actuator has a single orifice) or equal to the sum of the areas of the orifices (where the actuator has multiple orifices), determined at the narrowest part of each orifice in a plane perpendicular to the center axis of the orifice. For an actuator having a single orifice, the effective diameter of the orifice (D_{eff}) may be calculated as D_{eff} = 2 √ (areal π); for an actuator having multiple orifices, the effective diameter of the orifices may be calculated as D_{eff} = 2√ (Σareas / π).

"Epinephrine" as used herein, also known as adrenaline, refers to the compound having the following formula: 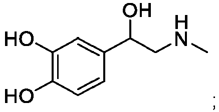 and having the name 4-(1-hydroxy-2-(methylamino)ethyl)benzene-1,2-diol. Epinephrine as used herein can be obtained from natural sources, such as, for example, from the adrenal glands of animals, or can be synthetically produced, such as, for example, from pyrocatechol. Epinephrine is a chiral molecule. The disclosed epinephrine formulations may comprise the (L)- or (D)-stereoisomers of epinephrine or a pharmaceutically acceptable salt of epinephrine, or a mixture of such stereoisomers (e.g., an optically active mixture or a racemic mixture). Preferably, the disclosed epinephrine formulations contain epinephrine or a pharmaceutically acceptable salt of epinephrine that substantially comprises the (L)-isomer, for example, at least about 70, 80, 90, or 95% of the epinephrine is the (L)-isomer. Epinephrine or a pharmaceutically acceptable salt of epinephrine is used in the devices and methods and uses described herein.

"Epinephrine formulation" as used herein refers to a formulation comprising epinephrine or a pharmaceutically acceptable salt of epinephrine.

"Laryngopharynx" and "laryngopharyngeal delivery" as used herein, refer respectively to the larynx and pharynx collectively and to delivery to the larynx and pharynx combined.

"Patient" as used herein, refers to a human patient.

"Pharmaceutically acceptable salt" as used herein, refers to those salts which are suitable for use in contact with the tissues of humans without undue toxicity, irritation, allergic response and the like, and are commensurate with a reasonable benefit/risk ratio. Pharmaceutically acceptable salts are well known in the art. For example, S. M. Berge et al., describe pharmaceutically acceptable salts in detail in J. Pharmaceutical Sciences, 1977, 66, 1-19. Pharmaceutically acceptable salts of epinephrine include those derived from suitable inorganic and organic acids. Examples of pharmaceutically acceptable, nontoxic acid addition salts are salts of an amino group formed with inorganic acids such as hydrochloric acid, hydrobromic acid, phosphoric acid, sulfuric acid and perchloric acid or with organic acids such as acetic acid, oxalic acid, maleic acid, tartaric acid, citric acid, succinic acid or malonic acid or by using other methods used in the art such as ion exchange. Other pharmaceutically acceptable salts include adipate, alginate, ascorbate, aspartate, benzenesulfonate, benzoate, bisulfate, bitartrate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxyethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, pivalate, propionate, stearate, succinate, sulfate, tartrate, thiocyanate, p-toluenesulfonate, undecanoate, valerate salts, and the like.

"Sectioned Alberta Idealized Throat" (S-AIT), as used herein, refers to an Adult Alberta Idealized Throat that is divided into sections having the dimensions depicted in FIG. 2. The Adult Alberta Idealized Throat is commercially available from Copley Scientific Ltd., Nottingham, U.K. (catalog # 8511). The AIT has been shown to be a physiologically representative model of the human throat that is predictive of deposition patterns in patients. McShane et al., Pulm. Pharmacol. & Therapeutics 50: 72-79 (2018); Sheth et al., Intl. J. Pharmaceutics 528: 360-371 (2017); Weers et al., J. Aerosol Med. and Pulmonary Drug Delivery 28: 1-13 (2015).

"Substantially dry," as it is used herein, refers to containing no more than about 10% liquid by weight. Preferably, the disclosed epinephrine particles contain no more than about 10% w/w liquid, for example, the particles can contain from about 1% to about 8%; from about 2% to about 6%; or from about 2% to about 5% w/w liquid based on the total weight of the particles.

"Therapeutically effective amount" as used herein refers to an amount of API (e.g., epinephrine or therapeutically acceptable salt thereof) that is sufficient to treat the stated disease, disorder, or condition or have the desired stated effect on the disease, disorder, or condition or one or more mechanisms underlying the disease, disorder, or condition in a human subject. In certain embodiments, when epinephrine is administered for the treatment of anaphylaxis, therapeutically effective amount refers an amount of epinephrine which, upon administration to a human, treats, or ameliorates or prevents anaphylaxis in the human, or exhibits a detectable therapeutic or preventative effect in the human having anaphylaxis.

"Treatment" as used herein refers to therapeutic applications associated with administering API (e.g., epinephrine) that ameliorate the indicated disease, disorder, or condition or one or more underlying mechanisms of said disease, disorder, or condition, including slowing or stopping progression of the disease, disorder or condition or one or more of the underlying mechanisms in a human subject. In certain embodiments, when epinephrine is administered for the treatment of anaphylaxis, treatment refers to therapeutic applications to slow or stop progression of anaphylaxis, prophylactic application to prevent development of anaphylaxis after potential exposure to an allergen, and/or reversal of anaphylaxis.

### 4.2. Detailed Description

Anaphylaxis often manifests as a serious and acute multi-system allergic reaction and is typically triggered by a cellular response to an allergen. Anaphylaxis often requires emergency room treatment and, if not treated properly and promptly, can result in death. Because the number of allergic reactions in the United States is progressively increasing, the incidence of anaphylaxis is also expected to increase. An attack of anaphylaxis may include angioedema manifested by swelling of the skin or other tissues, upper respiratory obstruction arising from swelling of the pharynx and/or larynx, and lower respiratory obstruction arising from bronchoconstriction. Anaphylaxis may also result in hypotension leading to anaphylactic shock.

In one embodiment, the present disclosure provides a metered dose inhaler (MDI), e.g. a pressured MDI, containing a formulation that comprises epinephrine or its pharmaceutically acceptable salt and that is suitable for administration to a patient in need thereof by inhalation, for example a patient suffering from anaphylaxis. The formulation employed in the present disclosure includes formulations comprising epinephrine or a pharmaceutically acceptable salt thereof found in over-the-counter MDIs, such as in MDI Primatene Mist^{™} (HFA) and other MDIs disclosed in the art, for example U.S. Patent Publ. No. 2005/061314. The disclosed MDI also comprises an actuator having one or more orifices with an effective diameter from about 0.12 mm to about 0.33 mm. In some embodiments, the MDI comprising the disclosed actuator reduces wasteful delivery of epinephrine to the oral cavity and increases delivery of epinephrine to target areas such as the larynx, pharynx, and lungs. Accordingly, the disclosed MDI having the disclosed actuator having one or more orifices with an effective diameter from about 0.12 mm to about 0.33 mm (e.g., 0.22 mm) decreases the number of administered doses (e.g., metered volumes of epinephrine) required to treat a patient in need of epinephrine (e.g., for treatment of anaphylaxis). Delivery of epinephrine to the larynx and pharynx is beneficial for treating or reducing the likelihood of upper airway obstruction in patients suffering from anaphylaxis. Delivery of epinephrine to the lungs is beneficial both for treating pulmonary manifestations of anaphylaxis, such as bronchospasm and pulmonary edema, and for achieving rapid absorption and systemic delivery of epinephrine. Delivery of epinephrine to the oral cavity is wasteful in that the oral cavity is not a target area for treatment of anaphylaxis (or conditions such as asthma) since the surface area for absorption in the oral cavity is small, which results in limited and slow systemic delivery. In addition, delivery of epinephrine to the oral cavity often results in an unpleasant taste that may discourage use of epinephrine by inhalation. In some embodiments, the disclosed MDI provides more reliable delivery of epinephrine to the larynx, pharynx and lungs by making delivery of epinephrine to these target areas less dependent on, or relatively independent of, the transverse or coaxial orientation of the MDI when a dose of epinephrine is administered. In some embodiments, the disclosed MDI provides the benefit of reducing the influence of MDI insertion angle, thereby eliminating the need for bulky and burdensome mouthpiece adaptors (e.g., a spacer or holding chamber) and thus making the disclosed MDI both easier to carry by a patient at risk of anaphylaxis and capable of reliably delivering epinephrine to target areas for treatment of anaphylaxis.

Use of a MDI containing a suspension comprising epinephrine or its pharmaceutically acceptable salt in combination with an actuator having one or more orifices with a narrow effective diameter, in accordance with the present invention, will likely lead to clogging of the actuator orifices after more than the recommended number of doses have been delivered. In some embodiments, e.g., where the MDI is a limited-dose MDI adapted for emergency use, the MDI provides up to 5 inhalation doses, up to 10 inhalation doses, up to 15 inhalation doses, up to 20 inhalation doses, up to 25 inhalation doses, up to 30 inhalation doses, up to 35 inhalation doses, up to 40 inhalation doses, up to 45 inhalation doses, or up to 50 inhalation doses. Such limited-dose MDIs are adapted to deliver short term treatment to a patient in need of epinephrine (e.g., for treatment of anaphylaxis) without clogging of the actuator orifice and preferably are bear or are accompanied by instructions stating the recommended number of doses.

The disclosed epinephrine formulation may be provided as a suspension formulation, including a pressurized suspension formulation that is suitable for aerosol delivery from the MDI. In certain instances, the suspension of epinephrine or a pharmaceutically acceptable salt thereof comprises epinephrine particles suspended in a liquefied propellant, such as a hydrofluoroalkane propellant, and a co-solvent as described herein. In certain aspects, the co-solvent is present in the formulation in an amount ranging from about 0.1% to about 4% w/w. The concentration of co-solvent contained in the MDI disclosed herein promotes the formation of an aerosolized plume or mist of epinephrine in fine particles that further increases delivery of epinephrine from the oral cavity to the laryngeal cavity, thereby increasing delivery of epinephrine to the lungs.

Without being limited to any particular theory, the disclosed actuator orifice diameter and co-solvent concentration each individually or in combination provide a MDI with the above mentioned performance characteristics that increases the delivery of epinephrine to the therapeutic areas of the larynx, pharynx and lungs, which decreases the number of doses (e.g., metered volumes of inhalation plumes) that need to be administered to achieve a therapeutic result.

### 4.2.1. Metered Dose Inhaler (MDI)

In some embodiments, the MDI is an over-the-counter MDI, such as MDI Primatene Mist^{™} (HFA) available from Armstrong Pharmaceuticals, Inc., a subsidiary of Amphastar Pharmaceuticals, that is modified by substituting a smaller actuator orifice. In some embodiments, the MDI is a MDI disclosed in U.S. Patent Publ. No. 2005/061314, modified by substituting a smaller actuator orifice. The disclosed MDI is preferably a pressure metered dose inhaler. In certain embodiments, the MDI comprises an actuator having one or more orifices that have an effective diameter of from about 0.12 mm to about 0.33 mm; from about 0.14 mm to about 0.33 mm; from about 0.16 mm to about 0.29 mm; from about 0.18 mm to about 0.27 mm; from about 0.20 mm to about 0.25 mm; or from about 0.21 mm to about 0.23 mm. In certain embodiments, the actuator orifice has an effective diameter of about 0.22 mm. In certain embodiments, the actuator orifice has an effective diameter of 0.22 mm. In some embodiments, the disclosed MDI is an orientation-independent MDI which reduces the influence of MDI insertion angle, thereby eliminating the need for bulky and burdensome mouthpiece adaptors.

### 4.2.2. API Formulation

The therapeutic formulation of the present disclosure comprises API (e.g., epinephrine or a pharmaceutically acceptable salt thereof) (i.e., an "API formulation"), preferably as a suspension. The API (e.g., epinephrine or salt thereof) is present in the formulation in an amount effective to exert the intended therapeutic action through delivery of one or more metered volumes of API (e.g., epinephrine) to the lungs. In some embodiments, the API (e.g., epinephrine or a pharmaceutically acceptable salt thereof) is present from about 0.1% to about 0.5%; from about 0.1% to about 0.4%; from about 0.2% to about 0.5%; or from about 0.2% to about 0.4% w/w based on the total weight of the formulation. In some embodiments, API (e.g., epinephrine or a pharmaceutically acceptable salt thereof) is present in about 0.10%, about 0.11%, about 0.12%, about 0.13%, about 0.14%, about 0.15%, about 0.16%, about 0.17%, about 0.18%, about 0.19%, about 0.20%, about 0.21%, about 0.22%, about 0.23%, about 0.24%, about 0.25%, about 0.26%, about 0.27%, about 0.28%, about 0.29%, about 0.30%, about 0.31%, about 0.32%, about 0.33%, about 0.34%, about 0.35%, about 0.36%, about 0.37%, about 0.38%, about 0.39%, about 0.40%, about 0.41%, about 0.42%, about 0.43%, about 0.44%, about 0.45%, about 0.46%, about 0.47%, about 0.48%, or about 0.49% w/w based on the total weight of the formulation. In some embodiments, the MDI containing a formulation comprising API (e.g., epinephrine or a pharmaceutically acceptable salt thereof) is capable of delivering an effective amount of API (e.g., epinephrine) to a patient in a single inhalation dose. The dose of API (e.g., epinephrine) delivered in a single inhalation can range from about 50 micrograms to several hundred milligrams. For example, the dose of API (e.g., epinephrine) delivered in a single inhalation using the present MDI can be about 50, 55, 60, 65, 70, 75, 80, 85, 90, 95, 100, 105, 110, 115, 120, 125, 130, 135, 140, 145, 150, 155, 160, 165, 170, 175, 180, 185, 190, 195, 200, 205, 210, 215, 220, 225, 230, 235, 240, 245, 250, 255, 260, 265, 270, 275, 280, 285, 290, 295, 300, 305, 310, 315, 320, 325, 330, 335, 340, 345, 350, 355, 360, 365, 370, 375, 380, 385, 390, 395, 400, 405, 410, 415, 420, 425, 430, 435, 440, 445, 450, 455, 460, 465, 470, 475, 480, 485, 490, 495, or about 500 micrograms, or the dose of API (e.g., epinephrine) delivered in a single inhalation using the present MDI can be about 0.5, 1, 15, 2, 2.5, 5, 7.5, 10, 15, 20, 25, 30, 40, 50, 100, 150, 200, 250, or about 300 milligrams.

The disclosed epinephrine formulations can comprise pharmaceutically acceptable salts of epinephrine, including, but not limited to, epinephrine hydrochloride or epinephrine bitartrate. Alternatively, the epinephrine formulations may comprise epinephrine free base. In some embodiment the epinephrine in the formulation consists of epinephrine free base. The disclosed epinephrine formulations may also comprise a mixture of two or more forms of epinephrine. The disclosed epinephrine formulations may also comprise one or more derivatives or analogs of epinephrine. The derivatives or analogs may be obtained from natural sources or from synthetic routes. Examples of derivatives or analogs of epinephrine include, but are not limited to, phenyl epinephrine and norepinephrine.

The disclosed API (e.g., epinephrine) formulations preferably contain API (e.g., epinephrine) in particle form. The disclosed API (e.g., epinephrine) formulations comprising API (e.g., epinephrine) in particle form are preferably biocompatible, and optionally are capable of affecting the rate of delivery of API (e.g., epinephrine). In some embodiments, the API (e.g., epinephrine) in particle form (e.g., epinephrine free base) is micronized. In some embodiments, the API (e.g., epinephrine) is present in the form of particles having a median diameter (e.g., mass median aerodynamic diameter) of 1 µm to 5 µm, preferably about 2 µm, optionally with a geometric standard deviation of 1-2, preferably 1.5-1.6. In some embodiments, the API (e.g., epinephrine) in particle form is obtained by spray drying.

In addition to API (e.g., epinephrine), the API (e.g., epinephrine) formulation can further include a variety of pharmaceutically acceptable excipients. In some embodiments, the API (e.g., epinephrine) formulation comprises API (e.g., epinephrine or a pharmaceutically acceptable salt thereof), and at least one pharmaceutically acceptable excipient. Both inorganic and organic materials can be used. Suitable materials can include, but are not limited to, lipids, phospholipids, fatty acids, inorganic salts, carboxylic acids, amino acids, carbohydrates, tartrate, and various sugars. In some embodiments, the API (e.g., epinephrine) particles are essentially, or substantially, free of liquid, that is, the particles are substantially dry.

The API (e.g., epinephrine) formulation of the present disclosure preferably comprises a liquefied propellant as the energy source to deliver API (e.g., epinephrine) to the lung. The liquefied propellant can be hydrofluoroalkane (HFA) propellant selected from 1,1,1,2-tetrafluoroethane, which is also known as HFA-134a, 1,1,1,2,3,3,3-heptafluoropropane, which is also known as HFA-227, 1,1-difluoroethane, which is also known as HFA-152a, and 1,3,3,3-tetrafluoropropene, which is also known as HFO-1234ze(E), or a mixture thereof. In some embodiments, the hydrofluoroalkane propellant is HFA-134a. In some embodiments, the liquefied propellant (e.g., HFA-134a) is present from about 95% to about 99.5% w/w, from about 96% to about 99% w/w, or from about 97% to about 99% w/w based on the total weight of the formulation. In certain embodiments, the liquefied propellant is present in about 97%, 98%, or 99%, particularly from about 98% to about 99% w/w based on the total weight of the formulation.

In some embodiments, the liquefied propellant of the present disclosure (or, if a co-solvent is present, of the propellant-cosolvent mixture) has a vapor pressure of about 3 bar to about 6 bar (absolute) at 20 °C. More specific representative vapor pressures include about 3.1 bar, about 3.2 bar, about 3.3 bar, about 3.4 bar, about 3.5 bar, about 3.6 bar, about 3.7 bar, about 3.8 bar, about 3.9 bar, about 4.0 bar, about 4.1 bar, about 4.2 bar, about 4.3 bar, about 4.4 bar, about 4.5 bar, about 4.6 bar, about 4.7 bar, about 4.8 bar, about 4.9 bar, about 5.0 bar, about 5.1 bar, about 5.2 bar, about 5.3 bar, about 5.4 bar, about 5.5 bar, about 5.6 bar, about 5.7 bar, about 5.8 bar, or about 5.9 bar (absolute) at 20 °C, or any range of vapor pressure created by using two of the aforementioned vapor pressures as endpoints. In some aspects, the liquefied propellant has a vapor pressure of about 3.7 bar to about 4.1 bar or about 5.5 bar to about 5.9 bar, most preferably about 5.7 bar (absolute) at 20 °C

In some embodiments, the surface tension of the liquified propellant (or, if a co-solvent is present, of the propellant-cosolvent mixture) is about 6 mN/m to about 9 mN/m at 20 _{°}C. More specific representative surface tensions include about 6.1 mN/m, about 6.2 mN/m, about 6.3 mN/m, about 6.4 mN/m, about 6.5 mN/m, about 6.6 mN/m, about 6.7 mN/m, about 6.8 mN/m, about 6.9 mN/m, about 7.0 mN/m, about 7.1 mN/m, about 7.2 mN/m, about 7.3 mN/m, about 7.4 mN/m, about 7.5 mN/m, about 7.6 mN/m, about 7.7 mN/m, about 7.8 mN/m, about 7.9 mN/m, about 8.0 mN/m, about 8.1 mN/m, about 8.2 mN/m, about 8.3 mN/m, about 8.4 mN/m, about 8.5 mN/m, about 8.6 mN/m, about 8.7 mN/m, about 8.8 mN/m, or about 8.9 mN/m at 20 °C, or any range of surface tension created by using two of the aforementioned surface tensions as endpoints. In some aspects, the surface tension of the liquefied propellant (or, if a co-solvent is present, of the propellant-cosolvent mixture) has a surface tension of about 6.8 mN/m to about 7.2 mN/m or about 8.5 mN/m to about 8.9 mN/m at 20 _{°}C.

A co-solvent is preferably included in the API (e.g., epinephrine) formulation of the present disclosure to improve the dispersion characteristics of the API (e.g., epinephrine) and also to help solubilize any surfactant that may be present. In certain aspects, the co-solvent promotes the formation of an aerosolized plume or mist of API (e.g., epinephrine) in fine particles that are able to reach the therapeutic region of the lungs upon delivery. In some embodiments, the co-solvent utilized in the formulation can be selected from ethanol, isopropanol, propylene glycol, ethylene glycol, propane, butane, isobutane, pentane, dimethyl ether, diethyl ether and the like, or mixtures thereof. In certain embodiments, the co-solvent is ethanol. The co-solvent (e.g., ethanol) can be present in the formulation in an amount ranging from about 0.1% to about 4% w/w, about 0.1% to about 3% w/w, or about 0.1% to about 2% w/w based on the total weight of the formulation. In certain embodiments, the co-solvent (e.g., ethanol) is present from about 0.5% w/w, about 1% w/w, or about 1.5% w/w based on the total weight of the formulation. In some embodiments, the co-solvent (e.g., ethanol) is present in 0.60%, 0.61%, 0.62%, 0.63%, 0.64%, 0.65%, 0.66%, 0.67%, 0.68%, 0.69%, 0.70%, 0.71%, 0.72%, 0.73%, 0.74%, 0.75%, 0.76%, 0.77%, 0.78%, 0.79%, 0.80%, 0.81%, 0.82%, 0.83%, 0.84%, 0.85%, 0.86%, 0.87%, 0.88%, 0.89%, 0.90%, 0.91%, 0.92%, 0.93%, 0.94%, 0.95%, 0.96%, 0.97%, 0.98%, 0.99%, 1.00%, 1.01%, 1.02%, 1.03%, 1.04%, 1.05%, 1.06%, 1.07%, 1.08%, 1.09%, 1.10%, 1.11%, 1.12%, 1.13%, 1.14%, 1.15%, 1.16%, 1.17%, 1.18%, 1.19%, 1.20%, 1.21%, 1.22%, 1.23%, 1.24%, 1.25%, 1.26%, 1.27%, 1.28%, 1.29%, 1.30%, 1.31%, 1.32%, 1.33%, 1.34%, 1.35%, 1.36%, 1.37%, 1.38%, 1.39%, or 1.40% w/w based on the total weight of the formulation. In certain aspects, the co-solvent (e.g., ethanol) is present in about 1.00% w/w based on the total weight of the formulation.

In certain embodiments, the API (e.g., epinephrine) formulation disclosed in the present disclosure has a neutral pH which allows for the use of a metal container and valve to eliminate any potential unsafe events, such as broken glass or even explosive containers, and also provides cost reduction and ease of manufacture.

In some embodiments, a surfactant may be added to the API (e.g., epinephrine) formulation to provide improved suspension properties. The surfactant can be selected from oleic acid, lecithin, and sorbitan oleates, e.g., sorbitan mono-oleate and sorbitan trioleate. In some embodiments, the surfactant is a sorbitan oleate such as polysorbate 80. The surfactant is included in the formulation to improve the physical stability of the formulation and ensure consistent delivery of medication from the MDI by coating the API (e.g., epinephrine) particles, which, in turn, prevents agglomeration of the particles, prevents adhesion of the particles to container walls, and provides lubrication for valve components in the MDI. In certain embodiments, the surfactant (e.g., polysorbate 80) is present in less than 0.05%, less than 0.03%, particularly about 0.02% w/w of the total weight of the formulation.

In some embodiments, an antioxidant may be added to the API (e.g., epinephrine) formulation. The antioxidant prevents oxidation of API (e.g., epinephrine), thereby increasing the stability of API (e.g., epinephrine) in the formulation. In some aspects, the antioxidant is soluble in the formulation. In some embodiments, the antioxidant is selected from thymol, tocopherol, ascorbyl palmitate, butylated hydroxyanisole, butylated hydroxytoluene, propyl gallate, sodium metabisulfite, citric acid, and sodium sulfite. In certain embodiments, the antioxidant is thymol. In certain embodiments, the antioxidant (e.g., thymol) is present in less than 0.05%, less than 0.03%, less than 0.02%, particularly about 0.01% w/w of the total weight of the formulation.

In some embodiments, the epinephrine formulation of the present disclosure comprises epinephrine, HFA 134a, ethanol, polysorbate 80, and thymol. In some embodiments, the epinephrine formulation comprises 0.19 to 0.48% w/w epinephrine, 98.49 to 98.78% w/w HFA 134a, 1% w/w ethanol, 0.02% w/w polysorbate 80, and 0.02% w/w thymol based on the total weight of the formulation. In some embodiments, the epinephrine formulation comprises 0.19% w/w epinephrine, 98.78% w/w HFA 134a, 1% w/w ethanol, 0.02% w/w polysorbate 80, and 0.01% w/w thymol based on the total weight of the formulation. In some embodiments, the epinephrine formulation comprises 0.27% w/w epinephrine, 98.70% w/w HFA 134a, 1% w/w ethanol, 0.02% w/w polysorbate 80, and 0.01% w/w thymol based on the total weight of the formulation. In some embodiments, the epinephrine formulation comprises 0.35% w/w epinephrine, 98.62% w/w HFA 134a, 1% w/w ethanol, 0.02% w/w polysorbate 80, and 0.01% w/w thymol based on the total weight of the formulation. In some embodiments, the epinephrine formulation comprises 0.48% w/w epinephrine, 98.49% w/w HFA 134a, 1% w/w ethanol, 0.02% w/w polysorbate 80, and 0.01% w/w thymol based on the total weight of the formulation.

### 4.2.3. Methods of Treatment

Also provided herein are methods of treatment and uses using the disclosed MDI. In some embodiments, a method of administering API (e.g., epinephrine) to a patient in need thereof using the disclosed MDI is provided. In some embodiments, an API (e.g., epinephrine) for use in administering to a patient in need thereof using the disclosed MDI is provided. In other embodiments, a method of treating a patient in need of API (e.g., epinephrine) using the disclosed MDI is provided. In some embodiments, an API (e.g., epinephrine) for use in treating a patient in need thereof using the disclosed MDI is provided. Additionally, a method for treating a patient in need of rescue therapy for an allergic reaction is contemplated. As such in another embodiment, the present disclosure provides a method of treating an allergic reaction in a patient in need thereof, comprising administering a therapeutically effective amount of API (e.g., epinephrine) using the disclosed MDI. In some embodiments, an API (e.g., epinephrine) for use in treating an acute allergic reaction in a patient in need thereof, comprising administering a therapeutically effective amount of API (e.g., epinephrine) using the disclosed MDI is provided. In some embodiments, the acute allergic reaction is grade 1 or higher as determined by the World Allergy Organization Subcutaneous Immunotherapy Systemic Reaction Grading System (the "WAO Grading System"). (Cox L. et al., J. Allergy Clin. Immunol. (2010) 125(3):569-74). In another embodiment, the acute allergic reaction is grade 2 or higher as determined by the WAO Grading System. In a further embodiment, the acute allergic reaction is grade 3 or higher as determined by the WAO Grading System. In a preferred embodiment, the patient is treated for anaphylaxis.

In some embodiments, the metered dose inhaler is an orientation independent metered dose inhaler and the administration using the orientation independent metered dose inhaler does not affect delivery of API (e.g., epinephrine) to the patient. In some embodiments, the proportion of the delivered API (e.g., epinephrine) dose that is administered to the larynx, pharynx and lungs using the disclosed metered dose inhaler is at least 90%, 95%, 96%, 97%, 98%, or 99% with the device at a transverse insertion angle, as compared with the proportion delivered to the larynx, pharynx and lungs with the device at a coaxial insertion angle, as determined by S-AIT testing at a flowrate of 30 L/min.

In some embodiments, the dose of epinephrine administered to the patient may be selected to correspond approximately to a dose sufficient to trigger muscular tremor in the patient. In some embodiments the epinephrine is administered shortly after the onset of an anaphylactic reaction, such as within five minutes, four minutes, three minutes, two minutes, or one minute after the onset of the reaction. In certain embodiments, a method of treating anaphylaxis or epinephrine for use in treating anaphylaxis in a patient comprises determining an approximate effective dose of inhalable epinephrine according to the method described herein; and administering the approximate effective dose of inhalable epinephrine to the patient after the onset of an anaphylactic reaction. In some embodiments, the epinephrine is administered a first time shortly after the onset of an anaphylactic reaction and again a second time if the anaphylactic reaction returns. Thus, in another embodiment, the present disclosure provides a method of treating an anaphylactic reaction or epinephrine for use in treating an anaphylactic reaction, comprising administering a therapeutically effective amount of epinephrine using the disclosed MDI (e.g. by administering 2 puffs) once every 1 to 10 minutes, such as once every 2, 3, 4, 5, 6, 7, or 8 minutes, until the anaphylactic reaction ameliorates. In a specific embodiment treatment comprises administering two puffs of epinephrine every 60 seconds until clinical resolution of allergic symptoms is obtained. In a preferred embodiment, treatment comprises administering two puffs of epinephrine periodically (e.g., every 60 seconds) until the patient shows a physiological response to the treatment (e.g., tachycardia, tremor, or both). Such a physiological response correlates closely with resolution of anaphylactic symptoms.

In some embodiments, the method of treatment and uses includes increasing delivery of API (e.g., epinephrine) to the larynx. In some embodiments, the disclosed MDI provides a laryngeal dose of API (e.g., epinephrine) that is from 1.6 times to 3.6 times greater than that provided by a MDI of the prior art (e.g., MDI Primatene Mist^{™} (HFA) having an actuator orifice diameter of about 0.42 mm). Also contemplated herein is a method of treating or reducing the likelihood of respiratory obstruction during an anaphylactic reaction that improves the treatment of anaphylaxis. As such in another embodiment, the present disclosure provides a method of treating or reducing the likelihood of respiratory obstruction during an anaphylactic reaction or epinephrine for use in treating or reducing the likelihood of respiratory obstruction during an anaphylactic reaction, comprising administering a therapeutically effective amount of epinephrine using the disclosed MDI. In some embodiments, the disclosed MDI provides a laryngopharyngeal dose of epinephrine that significantly improves the treatment of laryngopharyngeal edema. In another embodiment, the present disclosure provides a method of treating laryngopharyngeal edema during an anaphylactic reaction or epinephrine for use in treating laryngopharyngeal edema during an anaphylactic reaction, comprising administering a therapeutically effective amount of epinephrine using the disclosed MDI. In some embodiments, the method of treatment or use provides a rapid onset of action as determined by clinical assessment. In certain embodiments, the onset of action is less than 120 seconds, less than 90 seconds, or less than 60 seconds, preferably within 30 to 60 seconds after the treatment is administered, as determined by clinical assessment.

In some embodiments, the method of treatment or use includes increased delivery of API (e.g., epinephrine) to the lung (e.g., to the bronchi, bronchioles, and/or alveoli). In certain embodiments, the disclosed MDI provides a lung dose of greater than 20%, greater than 25%, greater than 30%, greater than 35%, greater than 40%, greater than 45%, or greater than 50% w/w of the total weight of API (e.g., epinephrine) in the dose of formulation administered, preferably greater than 40% w/w of the total weight of API (e.g., epinephrine) in the dose of formulation administered.

In some embodiments, the blood plasma concentrations of API (e.g., epinephrine) achieved using the present disclosed MDI are significantly less variable than API (e.g., epinephrine) administered by injection, thereby providing an advantage over intramuscular administration. In some embodiments, this decreased variability, i.e., greater reliability, in peak and time to peak systemic API (e.g., epinephrine) concentrations (Cₘₐₓ and Tₘₐₓ, respectively) through administration using the disclosed MDI results in greater consistency in therapeutic response and an improved safety profile. Moreover, the increased API (e.g., epinephrine) delivered to the lungs as particles in substantially dry form promotes rapid absorption and time to peak blood plasma concentrations, further improving the therapeutic benefits of API (e.g., epinephrine), for example, the ability of epinephrine to arrest a rapidly progressing anaphylactic reaction.

In certain embodiments, in addition to the above-mentioned benefits, the presently disclosed MDI having the disclosed actuator orifice with an effective diameter and co-solvent concentration provides an inhalable administration that increases the delivery of API (e.g., epinephrine) to the target areas of the larynx, pharynx, and lungs with decreased administration doses (e.g., metered volumes of inhalation plumes).

In another embodiment, kits are provided for the treatment of a patient in need of API (e.g., epinephrine for treatment of anaphylaxis). Such kits may comprise an MDI of the present disclosure and instructions for use. In some embodiments, a kit comprises an MDI in accordance with the present disclosure that is ready for use (e.g., the canister is installed in the actuator). In some embodiments, a kit comprises one or more actuators and one or more canisters in accordance with the present invention in a single package, where the canisters and actuators are separate parts and optionally may also comprise instructions for combining them and for use.

### 4.2.4. Numbered Embodiments

Embodiment 1. A kit for making a pressurized metered dose inhaler, the kit comprising:
a canister containing a formulation comprising an active pharmaceutical ingredient (API); and an actuator adapted to house the canister, the actuator having one or more orifices with an effective diameter of from about 0.12 mm to about 0.33 mm.

Embodiment 2. The kit of embodiment 1, wherein the API is in suspension.

Embodiment 3. The kit of embodiment 1 or embodiment 2, wherein the API is epinephrine or a pharmaceutically acceptable salt thereof.

Embodiment 4. The kit of any one of the preceding embodiments, wherein the effective diameter of the one or more orifices is from about 0.14 mm to about 0.31 mm.

Embodiment 5. The kit of embodiment 4, wherein the effective diameter of the one or more orifices is from about 0.16 mm to about 0.29 mm.

Embodiment 6. The kit of embodiment 5, wherein the effective diameter of the one or more orifices is from about 0.18 mm to about 0.27 mm.

Embodiment 7. The kit of embodiment 6, wherein the effective diameter of the one or more orifices is from about 0.20 mm to about 0.25 mm.

Embodiment 8. The kit of embodiment 7, wherein the effective diameter of the one or more orifices is from about 0.21 mm to about 0.23 mm.

Embodiment 9. The kit of embodiment 8, wherein the effective diameter of the one or more orifices is about 0.22 mm.

Embodiment 10. The kit of embodiment 9, wherein the effective diameter of the one or more orifices is 0.22 mm.

Embodiment 11. The kit of any one of embodiments 1-10, wherein the one or more orifices are circular.

Embodiment 12. The kit of any one of embodiments 1-10, wherein the one or more orifices comprise one or more peanut-, clover-, cross- or slot-shaped orifices.

Embodiment 13. The kit of any one of embodiments 1-12, wherein the actuator has a single orifice.

Embodiment 14. The kit of any one of embodiments 1-11, wherein the actuator has a plurality of orifices.

Embodiment 15. The kit of any one of embodiments 1-14, wherein the formulation further comprises:
a liquefied propellant; and
a co-solvent.

Embodiment 16. The kit of embodiment 15, wherein the co-solvent is present from about 0.1% to about 4% w/w based on the total weight of the formulation.

Embodiment 17. The kit of embodiment 16, wherein the co-solvent is present from about 0.1% to about 3% w/w based on the total weight of the formulation.

Embodiment 18. The kit of embodiment 17, wherein the co-solvent is present from about 0.1% to about 2% w/w based on the total weight of the formulation.

Embodiment 19. The kit of any one of embodiments 15-18, wherein the co-solvent is ethanol, isopropanol, propylene glycol, ethylene glycol, propane, butane, isobutene, pentane, dimethyl ether, diethyl ether, or a mixture thereof.

Embodiment 20. The kit of embodiment 19, wherein the co-solvent is ethanol.

Embodiment 21. The kit of any one of embodiments 15-20, wherein the liquefied propellant is present from about 95% to about 99.5% w/w based on the total weight of the formulation.

Embodiment 22. The kit of embodiment 21, wherein the liquefied propellant is present from about 96% to about 99% w/w based on the total weight of the formulation.

Embodiment 23. The kit of embodiment 22, wherein the liquefied propellant is present from about 97% to about 99% w/w based on the total weight of the formulation.

Embodiment 24. The kit of any one of embodiments 15-23, wherein the liquefied propellant has a vapor pressure of about 5.5 bar to about 5.9 bar (absolute), preferably about 5.7 bar (absolute) at 20 _{°}C.

Embodiment 25. The kit of any one of embodiments 15-23, wherein the liquefied propellant is 1,1,1,2-tetrafluoroethane (HFA-134A), 1,1,1,2,3,3,3-heptafluoropropane (HFA-227), or a mixture thereof.

Embodiment 26. The kit of embodiment 25, wherein the liquefied propellant is 1,1,1,2-tetrafluoroethane (HFA-134A).

Embodiment 27. The kit of any one of embodiments 15-26, wherein the API is present from about 0.1% to about 0.5% w/w based on the total weight of the formulation.

Embodiment 28. The kit of embodiment 27, wherein the API is present from about 0.1% to about 0.4% w/w based on the total weight of the formulation.

Embodiment 29. The kit of embodiment 28, wherein the API is present from about 0.1% to about 0.3% w/w based on the total weight of the formulation.

Embodiment 30. The kit of any one of embodiments 15-29, wherein the API is epinephrine free base.

Embodiment 31. The kit of any one of embodiments 15-30, wherein the formulation further comprises a surfactant.

Embodiment 32. The kit of embodiment 31, wherein the surfactant is selected from mono- or poly-sorbitan oleates, oleic acid, and lecithin.

Embodiment 33. The kit of any one of embodiments 15-21, wherein the formulation further comprises an antioxidant

Embodiment 34. The kit of embodiment 33, wherein the antioxidant is selected from thymol, tocopherol, ascorbyl palmitate, butylated hydroxyanisole, butylated hydroxytoluene, propyl gallate, citric acid, sodium metabisulfite, and sodium sulfite.

Embodiment 35. The kit of embodiment 15, wherein:
the API is epinephrine free base in suspension at a concentration of 1.9% w/w based on the total weight of the formulation;
the liquefied propellant is 1,1,1,2-tetrafluoroethane (HFA-134a);
the co-solvent is ethanol at about 1% w/w based on the total weight of the formulation; wherein the formulation further comprises
polysorbate 80; and
thymol,
and wherein the formulation is free of sulfites.

Embodiment 36. The kit of any one of embodiments 15-35, which is capable of delivering a dose of epinephrine or pharmaceutically acceptable salt thereof such that the proportion of the delivered dose that reaches the larynx, pharynx, and lungs when the metered dose inhaler is in a transverse orientation is at least 90%, preferably at least 95% of the proportion of the delivered dose that reaches the larynx, pharynx, and lungs when the metered dose inhaler is in an coaxial orientation, as determined with the Sectioned Alberta Idealized Throat at a flowrate of 30 L/min.

### 5. EXAMPLES

The following examples relate to the development of a novel treatment for anaphylaxis using aerosolized epinephrine. A sectioned Alberta Idealized Throat (S-AIT) device was built out of a photopolymer, RDG850 (VeroGray^{™}, Stratasys, Eden Prairie, MN) to allow for the estimation of deposition in three regions of interest within the extrathoracic airways (e.g., oral cavity, larynx, and upper trachea). The S-AIT showing these anatomical regions is shown in FIG. 2.

The S-AIT was used to evaluate deposition patterns obtained with commercial Primatene Mist HFA (a suspension MDI formulated with HFA-134a delivering micronized epinephrine; Amphastar Pharmaceutics) in delivering epinephrine to regions within the S-AIT and to a downstream filter. The lung dose (modeled by the filter dose) is an important metric in therapy of anaphylaxis both for treating bronchospasm and owing to a rapid uptake of epinephrine into the systemic circulation; while delivery to the larynx and pharynx is desirable for treating or reducing the likelihood of upper airway obstruction.

### Example 1: Deposition of Epinephrine with the Commercial Primatene Mist HFA MDI

Drug delivery was quantified by actuating MDIs directly into a filter to capture the dose of drug emitted from the device at a flowrate of 10, 30, 60 or 100 L/min using an experimental setup that is described in the United States Pharmacopeia. USP 44(5) General Chapter <601> Inhalation and Nasal Drug Products - Aerosols, Sprays, and Powders - Performance Quality Tests. 2019) with the following modifications. A bacterial/viral filter (VP7100 viral/bacterial filter, KEGO corporation) was used to capture the dose emitted by the MDI in place of a DUSA (dosage unit sampling apparatus) as specified in the USP. In both methods, the inhaler is actuated directly into the filter/DUSA at an inhalation flowrate of 30 L/min, with the MDI shaken for 5 seconds prior to each actuation, the MDI is actuated and held down for 1-2 seconds and the vacuum pump drawing air through the inhaler and filter/DUSA is turned off after 10 seconds, and the dose emitted by the MDI and depositing on the inner surfaces of the filter/DUSA is assayed with appropriate methods.

Three actuations were delivered during each test, corresponding to a nominal label claim of 375 µg epinephrine (3 x 125 µg) of the commercial formulation. 30 L/min is considered the ideal target inhalation flowrate for MDIs (e.g., Laube et al., Eur Respir J. 2011, 37(6):1308-31). Deposition patterns obtained with the commercial Primatene Mist HFA MDI were examined using a S-AIT and downstream filter. Tests were performed using two inhaler insertion angles to investigate the influence of inhaler orientation on deposition. The inhaler was oriented either (a) coaxially, in line with the axis of the oral cavity of the S-AIT at 29° from horizontal or (b) transversely, directed towards the "tongue" of the S-AIT along the horizontal axis of 0° (see FIG. 3), to determine the dependence of deposition on insertion angle.

### Quantifying Deposition

Prior to testing, the inner sections of the S-AIT were coated with silicone grease (Molykote 316; Dow Corning). After providing sufficient time for solvent to evaporate (~15 minutes), the six sections of the S-AIT were assembled, with vacuum grease (High Vacuum Grease, Dow Corning) applied between mating surfaces to create an air-tight seal. The mouthpiece adapter (either coaxial or transverse) was then fixed to the entrance of the S-AIT, while a filter (inhale 8 viral/bacterial filter; KEGO corporation) was placed downstream to capture the dose of drug escaping deposition in the extrathoracic region. A vacuum pump (RV5; Edwards) was used to generate airflow through the MDI, S-AIT, and downstream filter, with the target flowrate being set by a control valve and monitored with a flowmeter (Model 4043, TSI). A schematic of the experimental setup is shown in FIG. 4.

At the start of each test, the vacuum pump was turned on, and the flowrate set to the target value of 30 L/min (volumetric). The MDI was shaken vigorously for 3 to 5 seconds, then placed in the mouthpiece adapter and immediately actuated into the S-AIT. After 5 seconds, the MDI was removed from the mouthpiece adapter, and the process was repeated until three total actuations were delivered into the throat and filter. As the label claim of Primatene Mist HFA is 125 micrograms of epinephrine per dose, the nominal total label claim for each test was 375 micrograms.

The S-AIT and downstream filter were then disassembled and subjected to chemical assay via UV spectroscopy. The top, middle, and bottom sections of the S-AIT were each washed twice with 10, 10, and 5 mL of 0.1 N hydrochloric acid, respectively, while the filter was washed three times with 10 mL of 0.1 N hydrochloric acid. Corresponding drug masses in each sample were quantified via UV absorbance relative to standard at a maximum wavelength of 279 nm using a diode array UV-vis spectrophotometer (Cary 8454; Agilent). The sum of drug masses recovered from the top, middle, and bottom sections of the S-AIT, together with the downstream filter, equaled the total recovered dose for each test. Suspension MDIs are known to demonstrate variable drug delivery between actuations and over the lifetime of the canister (e.g., Hatley et al., Aerosol Med Pulm Drug Deliv. 2016, 30(1):71-9 and Chierici et al., Expert Opin Drug Deliv. 2020, 17(7):1025-39); *in vitro* deposition in each region of interest was thus normalized by the total recovered dose for each individual test to remove such sources of variability from results.

Prior to each test, the MDI canister was primed by firing a single shot to waste. Five repeated measures were obtained with each orientation (n = 5). The block of tests utilizing the coaxial insertion angle were performed first, followed by the block of tests utilizing the transverse insertion angle. Environmental conditions in the laboratory were monitored with a digital hygrometer/thermometer (MI70 Measurement Indicator with HMP75B Humidity and Temperature Probe; Vaisala); temperatures ranged from 22.4 to 23.8 °C, while relative humidity ranged from 4.1 to 21.3%.

### Results and Analysis

Deposition measured in each section of the S-AIT and the downstream filter (reflecting delivery to the lungs) at a flowrate of 10, 30, 60, and 100 L/min for the commercial Primatene MDI is summarized in **Table 1.** At 30 L/min, the average recovered dose ranged from 349.8 ± 40.9 µg (average ± standard deviation) for the coaxial orientation to 368.4 ± 12.9 µg for the transverse orientation. These average recovered doses, corresponding to 93.3 to 98.2% of the nominal label claim, indicated that total recovery was adequate.

**Table 1. Measured deposition of epinephrine in regions of interest expressed as a percentage of recovered dose at an inhalation flowrate of 10, 30, 60, and 100 L/min. Values reported as averages with standard deviations in parenthesis (n = 3). Averaged values across both inhaler orientations are also reported (for which n = 6).**

| **Inha-Iation Flowrate (L/min)** | **Insertion Angle** | **Recovered Dose (µg)** | **Oral Cavity Dose (% recov.)** | **Laryn-geal Dose (% recov.)** | **Upper Trachea Dose (% recov.)** | **Filter Dose (% recov.)** | **Laryn-geal + Filter dose (% recov.)** |
|---|---|---|---|---|---|---|---|
| 10 | Coaxial | 352.2 (9.7) | 46.7 (6.3) | 4.2 (2.5) | 2.2 (0.2) | 46.9 (4.7) | 51.2 (4.9) |
| | Transverse | 374.6 (12.0) | 69.7 (4.0) | 4.3 (0.1) | 1.9 (0.1) | 24.1 (2.7) | 28.4 (2.7) |
| | Averaged | 363.4 (15.7) | 58.2 (13.1) | 4.3 (1.6) | 2.0 (0.2) | 35.5 (13.0) | 39.8 (13.0) |
| 30 | Coaxial | 349.8 (40.9) | 33.2 (5.4) | 5.0 (0.4) | 2.9 (1.6) | 58.9 (0.5) | 64.0 (0.8) |
| | Transverse | 368.4 (12.9) | 57.0 (5.1) | 7.6 (2.9) | 2.8 (1.1) | 32.6 (8.4) | 40.2 (5.7) |
| | Averaged | 359.1 (29.0) | 45.1 (13.5) | 6.3 (2.4) | 2.9 (1.2) | 45.8 (15.4) | 52.1 (13.5) |
| 60 | Coaxial | 366.5 (22.2) | 28.5 (6.5) | 10.9 (2.3) | 4.4 (0.7) | 56.3 (3.9) | 67.2 (5.3) |
| | Transverse | 375.8 (17.2) | 42.3 (6.3) | 8.8 (1.6) | 2.6 (1.3) | 46.2 (7.3) | 55.0 (8.1) |
| | Averaged | 371.2 (18.4) | 35.4 (9.6) | 9.8 (2.1) | 3.5 (1.4) | 51.3 (7.6) | 61.1 (9.1) |
| 100 | Coaxial | 338.6 (20.1) | 26.6 (2.7) | 17.5 (1.0) | 5.3 (0.8) | 50.4 (5.0) | 68.0 (4.2) |
| | Transverse | 332.9 (28.2) | 26.1 (2.9) | 10.0 (1.3) | 2.8 (1.7) | 61.1 (3.1) | 71.1 (2.6) |
| | Averaged | 335.7 (22.2) | 26.4 (2.6) | 13.8 (4.3) | 4.1 (1.8) | 55.7 (6.9) | 69.5 (3.6) |

The proportion of the delivered epinephrine dose that results in laryngeal and filter deposition is dependent on the insertion angle of the device. As the flowrate is increased from 10 L/min to 100 L/min, the dependency of the laryngeal and filter dose deposits on the insertion angle decreases linearly (51.2% coaxial to 28.4% transverse; 64.0% coaxial to 40.2% transverse; 67.2% coaxial to 55.0% transverse; and 68.0% coaxial to 71.1% transverse) with a linear increase in the average combined laryngeal and filter dose deposits (39.8%, 52.1%, 61.1%, and 69.5%).

At 30 L/min, approximately 30-50% of the dose deposits in the oral cavity (33.2% coaxial, 57.0% transverse), and a considerable dependence on the insertion angle is observed in the laryngeal dose (5.0% coaxial vs. 7.6% transverse), the filter dose (58.9% coaxial vs 32.6% transverse), and the total dose delivered to these therapeutic target areas (64.0% coaxial vs. 40.2% transverse).

### Example 2: Influence of Actuator Orifice Diameter on Deposition at 30 L/min

Deposition patterns obtained with Primatene Mist HFA delivered from MDI actuators with various orifice diameters were compared using a S-AIT and downstream filter, as described in Example 1. MDI actuators with various orifice diameters were tested. Three actuator orifice diameters, including 0.42, 0.33, and 0.22 mm (part numbers 10028865 = BK631, 10016813 = BK632, and 10016819 = BK633, respectively; Bespak, Consort Medical), were selected for testing out of a larger set of five nominal diameters (including 0.48, 0.42, 0.33, 0.30, and 0.22 mm). Primatene Mist HFA canisters from lot numbers PR302D8 and PR303E8 were used during testing. Coaxial tests were performed with a canister from lot number PR302D8. Transverse tests were performed with a canister from lot number PR303E8. After each day of testing, actuators were cleaned with warm soapy tap water, then rinsed with DIUF water and dried with compressed building air.

Prior to each day of testing, each MDI canister was primed by firing two shots to waste. No difference between total deposition was observed in comparison to priming with a single shot to waste before each test (e.g., total recovered dose was ~360 µg for both priming methods at the commercial orifice diameter in **Table 1** and **Table 2).** Testing order for the three actuator orifice diameters was randomized. Five repeated measures were obtained with each actuator orifice diameter (n = 5). The block of tests utilizing the coaxial insertion angle were performed first, followed by the block of tests utilizing the transverse insertion angle. Environmental conditions in the laboratory as monitored with a digital hygrometer/thermometer (MI70 Measurement Indicator with HMP75B Humidity and Temperature Probe; Vaisala) showed that temperatures ranged from 22 to 25 °C, while relative humidity ranged from 4 to 20%.

In the first series of tests, deposition patterns were evaluated at a flowrate of 30 L/min for each of the three actuators. Tests were performed using the inhaler oriented either coaxially or transversely.

### Results and Analysis

Deposition measured in each section of the S-AIT and the downstream filter at a flowrate of 30 L/min for the chosen actuator orifice diameters is summarized in **Table 2.** FIG. 5 and FIG. 6 providing additional detail on deposition obtained with coaxial and transverse insertion angles, respectively, while FIG. 7 compares deposition in each region of interest with coaxial and transverse orientations for each orifice diameter. FIG. 8 shows the total deposition to the laryngopharynx and filter at the tested orifice diameters. The average recovered dose ranged from 298.3 ± 18.9 µg (average ± standard deviation) for the 0.22 mm orifice in the coaxial orientation to 378.8 ± 35.7 µg for the 0.42 mm orifice in the transverse orientation. These average recovered doses, corresponding to 79.5% to 101.0% of the nominal label claim, indicated that total recovery was adequate.

**Table 2. Measured deposition of epinephrine in regions of interest expressed as a percentage of recovered dose for various actuator orifice diameters at an inhalation flowrate of 30 L/min. Values reported as averages with standard deviations in parenthesis (n = 5).**

| **Actuator Orifice Diameter (mm)** | **Insertion Angle** | **Recovered Dose (µg)** | **Oral Cavity Dose (% recov.)** | **Laryngeal Dose (% recov.)** | **Upper Trachea Dose (% recov.)** | **Filter Dose (% recov.)** | **Laryngeal + Filter dose (% recov.)** |
|---|---|---|---|---|---|---|---|
| 0.42 | Coaxial | 343.5 (39.2) | 42.4 (4.8) | 25.9 (2.3) | 3.1 (0.9) | 28.6 (5.8) | 54.5 (4.6) |
| | Transverse | 378.7 (35.7) | 54.3 (8.0) | 16.0 (5.3) | 2.8 (0.8) | 26.9 (6.7) | 42.9 (8.0) |
| | Average | 361.1 (39.9) | 48.3 (8.8) | 21.0 (6.5) | 3.0 (0.8) | 27.8 (6.0) | 48.7 (8.7) |
| 0.33 | Coaxial | 318.8 (22.3) | 44.1 (8.9) | 23.7 (1.6) | 4.5 (0.7) | 27.7 (8.7) | 51.4 (9.4) |
| | Transverse | 343.7 (23.7) | 44.8 (4.5) | 23.6 (2.7) | 3.2 (1.5) | 28.3 (4.2) | 51.9 (4.5) |
| | Average | 331.2 (25.4) | 44.5 (6.7) | 23.7 (2.1) | 3.8 (1.3) | 28.0 (6.4) | 51.7 (7.0) |
| 0.22 | Coaxial | 298.3 (18.9) | 26.0 (7.4) | 26.6 (2.6) | 4.6 (1.4) | 42.7 (9.0) | 69.4 (7.7) |
| | Transverse | 341.9 (26.9) | 28.8 (4.2) | 24.2 (1.8) | 4.7 (1.6) | 42.3 (6.9) | 66.5 (5.5) |
| | Average | 320.1 (31.8) | 27.4 (5.9) | 25.4 (2.4) | 4.6 (1.4) | 42.5 (7.6) | 68.0 (6.5) |

For the largest actuator orifice, 0.42 mm, approximately 50% of the dose deposits in the oral cavity (42.4% coaxial, 54.3% transverse), and a considerable dependence on the insertion angle is observed in the laryngeal dose (25.9% coaxial vs 16.0% transverse) and in the combined laryngeal and filter dose (54.5% coaxial vs 42.9% transverse). With the 0.33 mm orifice diameter, the dependence of the laryngeal dose and oral cavity dose on insertion angle is reduced, though the oral cavity dose (~ 44%) and filter dose (~ 28%) are similar to the averages obtained with the larger 0.42 mm orifice diameter and a coaxial insertion angle and the combined laryngeal and filter dose is essentially independent of the insertion angle (51.4% coaxial vs 51.9% transverse). For the smallest orifice diameter of 0.22 mm, deposition in all regions of interest appears essentially independent of inhaler orientation. Deposition in the oral cavity was reduced considerably to approximately 27.5% of the recovered dose, while the filter dose increased to ~ 42% (most evident in FIG. 7). The combined laryngeal and filter dose was increased and essentially independent of inhaler orientation (69.4% coaxial vs 66.5% transverse).

Several conclusions are supported by this data. First, compared to the 0.42 mm orifice diameter, smaller orifice diameters of 0.33 mm and 0.22 mm provide more consistent deposition in target regions of therapeutic interest with regards to inhaler insertion angle. Second, use of the 0.22 mm orifice diameter diminishes delivery to the oral cavity. Third, use of the 0.22 mm orifice diameter increased delivery to the lungs while maintaining the proportion of the dose delivered to the larygopharyn geal region, thereby increasing delivery to therapeutic regions of interest (a laryngeal dose of ~ 25% of the total recovered dose was observed at an inhalation flowrate of 30 L/min).

Considering the S-AIT as analogous to the extrathoracic region *in vivo,* these results suggest that the commercial Primatene Mist HFA canister can be modified by using a smaller actuator orifice (e.g. 0.22 mm) in place of the larger actuator orifice in commercially available Primatene Mist HFA MDIs to reduce the dependence of inhaler performance on insertion angle, maintain a high dose to the larynx, and increase the dose delivered to the lungs by shifting deposition from the oral cavity to the lungs. Without being bound by theory, the inventors believe that these effects are caused in part by a reduction in the momentum of the spray emitted from the MDI when an actuator with a smaller orifice diameter is used, thereby reducing deposition in the oral cavity. Given these favorable shifts in deposition, the 0.22 mm actuator orifice was selected as a good candidate for further testing across a range of flowrates, as discussed in the next section.

### Example 3: Deposition Behavior at Various Flowrates for the 0.22 mm Orifice Diameter

Deposition of Primatene Mist HFA delivered with a 0.22 mm diameter actuator orifice was quantified in the S-AIT and downstream filter at a number of inhalation flowrates of interest, including 10, 30, 60, and 100 L/min. Methods used to quantify deposition were identical to those described in the previous section (see FIG. 4), with the inhalation flowrate altered to the value of interest. The environmental conditions were measured using a digital hygrometer/thermometer; temperatures ranged from 21.5 to 23.5 °C, while relative humidity ranged from 40 to 65%. The nominal label claim for each experiment was again 375 µg (3 x 125 µg) epinephrine. Canisters from lot numbers PR302D8 and PR303E8 were again used in this round of testing.

Deposition was first measured at a flowrate of 30 L/min for both the coaxial and transverse insertion angles, followed by 60, 100, 10, and an additional set of experiments at 30 L/min. Three repeated measures were taken at the inhalation flowrates of 10, 60, and 100 L/min (n = 3). For 30 L/min, six repeated measures were taken and data was collated with that obtained in the previous section for 30 L/min and the 0.22 mm actuator orifice diameter (wherein five repeated measures were taken), for a total of eleven repeated measures (n = 11). Environmental conditions were again measured using a hygrometer/thermometer; temperature ranged from 21.5 to 23.5 °C, while relative humidity ranged from 40 to 65%.

### Results and Analysis

Deposition measured in each section of the S-AIT and the downstream filter using the 0.22 mm actuator orifice at flowrates of interest is summarized in **Table 3.** FIG. 9 and FIG. 10 provide additional detail on deposition obtained with the coaxial and transverse insertion angles, respectively, while FIG. 11 compares deposition in each region of interest with coaxial and transverse orientations across flowrates of interest. FIG. 12 shows the total deposition to the larynx and filter at the tested orifice diameters.

The recovered dose ranged from 241.1 ± 26.0 µg for the transverse orientation at 10 L/min to 356.2 ± 30.8 µg for the transverse orientation at 30 L/min. This wider variability in recovered dose than was observed in the first set of experiments as described above likely related to the use of canisters at higher dose counts. For the 30, 60, and 100 L/min flowrates, the laryngeal dose lies consistently between 19 and 26% of the recovered dose regardless of inhaler orientation (equivalent to between 71 and 98 µg epinephrine for a 375 µg dose). At 10 L/min, the laryngeal dose is roughly halved to ~12.5% of the recovered dose. The filter dose, at all flowrates, fell between 40 and 58% of the recovered dose.

**Table 3. Measured deposition of epinephrine in regions of interest expressed as a percentage of recovered dose for the 0.22 mm actuator orifice diameter at various inhalation flowrates. Values reported as averages with standard deviations in parenthesis (n = 3). Averaged values across both inhaler orientations are also reported (for which n = 6).**

| **Inhalation Flowrate (L/min)** | **Insertion Angle** | **Recovered Dose (µg)** | **Oral Cavity Dose (% recov.)** | **Laryn-geal Dose (% recov.)** | **Upper Trachea Dose (% recov.)** | **Filter Dose (% recov.)** | **Laryngeal + Filter dose (% recov.)** |
|---|---|---|---|---|---|---|---|
| 10 | Coaxial | 265.8 (23.2) | 32.2 (2.3) | 13.2 (0.9) | 2.9 (1.5) | 51.7 (2.9) | 64.9 (2.1) |
| | Transverse | 241.1 (26.0) | 37.2 (3.6) | 12.1 (0.9) | 2.9 (0.7) | 47.8 (2.7) | 59.9 (3.2) |
| | Averaged | 253.5 (25.9) | 34.7 (3.9) | 12.7 (1.0) | 2.9 (1.1) | 49.7 (3.3) | 62.4 (3.7) |
| 30* | Coaxial | 313.2 (37.5) | 29.0 (6.4) | 25.4 (4.7) | 4.8 (1.8) | 40.9 (9.0) | 66.2 (6.9) |
| | Transverse | 356.2 (30.8) | 31.6 (5.3) | 19.9 (6.0) | 3.8 (2.0) | 44.8 (9.9) | 64.6 (6.3) |
| | Averaged | 334.7 (40.1) | 30.3 (5.9) | 22.6 (6.0) | 4.3 (1.9) | 42.8 (9.4) | 65.4 (6.5) |
| 60 | Coaxial | 328.5 (19.8) | 24.1 (1.5) | 20.1 (1.2) | 2.8 (0.5) | 53.1 (2.1) | 73.1 (2.0) |
| | Transverse | 340.3 (27.6) | 18.6 (3.7) | 20.9 (9.1) | 3.3 (0.5) | 57.3 (11.4) | 78.1 (3.3) |
| | Averaged | 334.4 (22.4) | 21.3 (3.9) | 20.5 (5.8) | 3.1 (0.5) | 55.2 (7.7) | 75.6 (3.7) |
| 100 | Coaxial | 302.8 (22.8) | 23.3 (1.4) | 23.3 (1.2) | 4.6 (0.8) | 48.7 (2.6) | 72.1 (1.9) |
| | Transverse | 271.4 (19.4) | 16.4 (3.1) | 21.3 (0.6) | 4.9 (2.7) | 57.4 (1.1) | 78.7 (0.5) |
| | Averaged | 287.1 (25.6) | 19.8 (4.4) | 22.3 (1.4) | 4.8 (1.8) | 53.0 (5.1) | 75.4 (3.9) |

| | | | | | | | |
|---|---|---|---|---|---|---|---|
| * n = 11 for the data at 30 L/min (n = 22 for average of the two orientations). | | | | | | | |

FIGs. 13-16 compare deposition in each region of the interest from the commercial Primatene Mist HFA actuator with the 0.22 mm actuator at inhalation flowrates of 10, 30, 60, and 100 L/min, respectively.

FIG. 17, FIG. 18, and FIG. 19 show the effect of the 0.22 mm actuator orifice on deposition in the larynx, in the filter, and in both the larynx and filter. FIG. 17 shows that the 0.22 mm actuator delivers a considerably larger dose to the larynx than the commercial actuator at all flowrates; considering averaged deposition across both inhaler orientations, the 0.22 mm orifice delivers anywhere between 1.6 times (at 100 L/min) and 3.6 times (at 30 L/min) as much epinephrine to the larynx as the commercial actuator. FIG. 18 shows that filter deposition remains high with the 0.22 mm actuator orifice (above 40% for all flowrates and inhaler orientations), with less variability in deposition occurring between the coaxial and transverse orientations than with the commercial actuator, particularly at the lower flowrates of 10, 30, and 60 L/min. FIG. 19 shows the overall increased deposition using the 0.22 mm actuator to the larynx and filter (e.g., desired therapeutic regions).

Moving from 10 L/min to 100 L/min, less dependence of insertion angle for the laryngeal and filter dose deposits was observed in comparison to the commercial actuator in **Table 1** (64.9% coaxial to 59.9% transverse; 66.2% coaxial to 64.6% transverse; 73.1% coaxial to 78.1% transverse; and 72.1% coaxial to 78.7% transverse) while higher total laryngeal and filter dose deposits were observed at all flowrates (62.4% versus 39.8%, 65.4% versus 52.1%, 75.6% versus 61.1%, and 75.4 versus 69.5%). At flowrates of 30 L/min and above, the combined laryngeal and filter dose was essentially independent of the insertion angle.

### Example 4: Measurement of Particle Size Distributions and Predictions of Regional Lung Deposition of Epinephrine Delivered with the 0.22 mm Actuator

Particle size distributions of epinephrine delivered with the 0.22 mm actuator were performed using the experimental setup shown in FIG. 20. Briefly, a Next Generation Impactor (Model 170 NGI; Copley) was used to capture dose exiting a regular non-sectioned AIT (used here to minimize assay steps) at an inhalation flowrate of 30 L/min. Interior surfaces of the AIT and the impaction plates within the NGI were coated with silicone grease (Molykote 316; Dow Corning) prior to testing. Inhalers were handled and actuated in accordance with the methods described previously in this report, with three actuations used per test (nominal label claim of 375 µg epinephrine). Following actuation, the AIT was washed twice with 10 mL of 0.1 N HCI, while each plate of the NGI was washed once with 5 mL of 0.1 N HCl. Samples were assayed via UV spectroscopy at the absorbance maximum of 279 nm, as discussed above.

Tests were performed using the 0.22 mm actuator orifice with Primatene Mist HFA. Both transverse and coaxial insertion angles were used, with three repeated measures taken at each orientation. Particle size distributions were characterized via calculation of the mass median aerodynamic diameter (MMAD) and geometric standard deviation (GSD) via linear interpolation (e.g., Hinds WC. 2nd ed. Hoboken, NJ: Wiley; 1999). A validated regional lung deposition model (e.g., Javaheri et al., J Aerosol Sci. 2013, 64:81-93 and Finlay et al., Lung Delivery of Aerosolized Dextran. 2000, 161:91-7) was used to estimate tracheobronchial and alveolar deposition from the measured particle size distributions under a simulated breath with an inhaled volume of 3.0 L, inhalation flowrate of 30 L/min, a pause between inhalation of (i) 0 or (ii) 10 seconds, and exhalation flowrate of 30 L/min. The density of epinephrine was taken as 1.283 g/cm³, air density was taken as 1.2 kg/m³, and the dynamic viscosity of air was taken as 1.85 × 10⁵ kg m⁻¹ s⁻¹.

### Results

**Table 4** summarizes measurements in the NGI obtained with the 0.22 mm actuator orifice. The distribution obtained with this orifice, with an MMAD of approximately 2 µm and a GSD of ~ 1.63, appeared unaffected by the insertion angle. **Table 5** shows predicted total lung dose and the distribution of deposition between the tracheobronchial airways and the alveolar region for pauses between inhalation and exhalation of 0 and 10 seconds. For a given breath hold, results differ negligibly for both insertion angles. For no breath hold, the total lung dose is approximately 212 µg, with ~ 37 µg depositing in the tracheobronchial airways and the remaining ~ 175 µg depositing in the alveolar region. For a 10 second breath hold, the total lung dose increases considerably to ~ 252 µg, caused by increased deposition in the alveolar region to ~ 213 µg (the tracheobronchial dose, at ~ 39 µg shows a negligible increase compared to the no breath hold case).

**Table 4. Measured deposition in the NGI using the 0.22 mm actuator orifice, together with previously reported data for the commercial actuator. Average with standard deviation in parenthesis. MMAD = mass median aerodynamic diameter, GSD = geometric standard deviation.**

| **Deposition (µg)** | **Actuator / Insertion Angle** | | | |
|---|---|---|---|---|
| | **0.22 mm Coaxial** | **0.22 mm Transverse** | **Commercial* Coaxial** | **Commercial* Transverse** |
| **Total Recovered** | 345.2 (25.8) | 363.0 (22.8) | 364.3 (47.4) | 418.8 (31.3) |
| **Throat** | 78.6 (12.7) | 95.8 (16.7) | 136.3 (24.7) | 212.9 (9.4) |
| **Stage 1** | 1.9 (0.2) | 1.5 (0.7) | 2.1 (0.9) | 0.8 (0.5) |
| **Stage 2** | 1.9 (1.2) | 1.4 (0.8) | 2.5 (0.4) | 1.1 (0.4) |
| **Stage 3** | 8.5 (1.5) | 8.8 (1.0) | 11.1 (3.0) | 6.8 (1.6) |
| **Stage 4** | 82.6 (7.0) | 80.8 (3.5) | 72.9 (15.2) | 56.0 (10.9) |
| **Stage 5** | 122.2 (7.7) | 121.7 (1.2) | 99.1 (14.8) | 96.8 (10.8) |
| **Stage 6** | 42.0 (1.6) | 43.6 (0.7) | 34.1 (3.5) | 37.7 (3.9) |
| **Stage 7** | 7.6 (0.8) | 9.5 (1.7) | 6.3 (0.5) | 6.6 (0.7) |
| **Stage 8** | 0.0 (0.0) | 0.0 (0.0) | 0.0 (0.0) | 0.0 (0.0) |
| **MMAD (µm)** | 2.00 | 1.98 | 2.05 | 1.93 |
| **GSD** (-) | 1.63 | 1.64 | 1.64 | 1.65 |

| | | | | |
|---|---|---|---|---|
| * Deposition previously reported for commercial actuator; MMAD and GSD calculated here for the commercial product using linear interpolation. | | | | |

**Table 5. Predicted regional lung deposition for the 0.22 mm actuator for an inhalation at 30 L/min, an inhaled volume of 3.0 L, and an exhalation at 30 L/min.**

| **Insertion Angle** | **Coaxial** | | **Transverse** | |
|---|---|---|---|---|
| **Pause between Inhalation and Exhalation (s)** | 0 | 10 | 0 | 10 |
| **Total Lung Dose (µg)** | 212.0 | 251.5 | 211.8 | 251.9 |
| **Tracheobronchial Dose (µg)** | 37.7 | 39.1 | 37.0 | 38.3 |
| **Alveolar Dose (µg)** | 174.3 | 212.4 | 174.8 | 213.6 |

These results indicate that a majority of the dose entering the lungs would likely deposit within the alveolar region given the small particle size distribution of epinephrine measured downstream of the AIT. A longer breath hold would likely increase the dose depositing in the lungs via an increase in the alveolar dose. For a given breath hold duration, results were consistent regardless of the inhaler insertion angle, indicating that the 0.22 mm may provide a fairly robust platform for delivering epinephrine across a reasonable range of insertion angles.

### Summary

Four studies were undertaken to evaluate the feasibility of various approaches in optimizing the deposition of aerosolized epinephrine for the treatment of anaphylaxis. In the first study, the delivery of epinephrine by the commercial Primatene Mist HFA MDI to various regions of interest was examined using an S-AIT and downstream filter at an inhalation flowrate of 30 L/min. In the second study, it was shown that the use of MDI actuators with smaller orifices than the commercial Primatene Mist HFA actuator could greatly influence regional deposition within an S-AIT and downstream filter at an inhalation flowrate of 30 L/min. Laryngeal deposition increased notably at the smallest orifice size of 0.22 mm, while filter deposition remained high. The small orifice provided a further benefit of reducing the influence of inhaler insertion angle on deposition. In the third study, testing with the 0.22 mm orifice across a range of flowrates (10, 30, 60, and 100 L/min) confirmed that the laryngeal dose was consistently higher than that achieved with the commercial Primatene Mist HFA MDI (between 1.6 and 3.6 times greater), while the filter dose remained high (greater than 40% of the recovered dose). Deposition patterns also showed a reduced dependence on inhaler insertion angle at flowrates of 10 L/min and above (e.g., 10, 30, and 60 L/min). In the fourth study, particle size distributions and modeling of regional lung deposition suggested that most of the dose reaching the lungs is expected to deposit in the alveolar region, and that breath holds between inhalation and exhalation may provide a means of enhancing the alveolar dose and the total lung dose. Overall, these results suggest that the use of a smaller orifice actuator (e.g., an actuator with a 0.22 mm diameter orifice) provides a good candidate for optimizing the deposition of aerosolized epinephrine.

### Example 5: Oral Inhaled Epinephrine for Treatment of Anaphylaxis

A clinical study was conducted to evaluate the safety, tolerability and efficacy of oral inhaled epinephrine treatment in patients experiencing a grade 2 or higher acute allergic reaction, as determined by the WAO Grading System, in a doctor's office setting upon exposure to an antigen by oral or injected challenges with food, drugs, allergy extracts or vaccines. Institutional Review Board approval was obtained for the study and informed consent was obtained from study participants. Study participants developing in-office acute allergic reactions were treated with metabisulfite-free epinephrine (Primatene Mist HFA, Amphastar Pharmaceuticals) delivered by oral inhalation via a Bespak BK633 actuator having a single orifice of diameter 0.22 mm. One to four doses of oral inhaled epinephrine were administered, spaced 60 seconds apart, until complete clinical resolution of symptoms was obtained. This development of a physiological response to treatment (increased heart rate, tremor or both) correlated closely with, and generally coincided with, complete resolution of acute allergic reaction symptoms. Any recurrence of allergic symptoms was treated with additional doses of oral inhaled epinephrine. Each dose consisted of two puffs providing a nominal dose of 125 mg epinephrine per puff. At the discretion of the investigator, 0.3 mg epinephrine could be administered by intramuscular injection as standard care at any point if the clinical response to oral inhaled epinephrine was judged to be inadequate.

Participants were monitored for physiologic response to epinephrine treatment (e.g., patient's subjective awareness of body or hand tremor, patient's subjective awareness of increased heart rate), timing of physiologic response after initiation of therapy, complete resolution of allergic symptoms and time to complete resolution of clinical symptoms after initiation of therapy. **Table 6** shows the patient results of the clinical study and **Table 7** shows the patient symptoms.

**Table 6. Clinical Study Results**

| **Patient** | **Age, Sex** | **Time to Onset of Allergic Reaction After Allergen Challenge** | **Puffs Given** | **Tachycardia** | **Tremors** | **Time to Resolution of Allergic Reaction After Treatment Initiated** |
|---|---|---|---|---|---|---|
| 1 | 21 F | 1 min | 4 | Yes | Yes | 3 mins |
| 2 | 52 F | 5 mins | 4 | Yes | Yes | within 2 mins |
| 3 | 41 F | 5 mins | 4 | Yes | Yes | 30 secs |
| 4 | 86 F | 1 min | 2 | Yes | Yes | 1 min |
| 5 | 66 F | 4 mins | 6 | Yes | Yes | 6 mins |
| 6 | 49 F | 2 mins | 9 | Yes | Yes | resolved after 5 puffs, but recurred after 5 mins |
| 7 | 41 F | 5 mins | 2 | Yes | Yes | 2 mins |
| 8 | 31 M | within 5 mins | 2 | Yes | Yes | 1 min |
| 9 | 71 F | 5 mins | 2 | Yes | Yes | 1 min |
| 10 | 32 F | 5 mins | 5 | Yes | Yes | 1 min |
| 11 | 25 F | | 2 | Yes | Yes | |
| 12 | 40 F | within 5 mins | 2 | Yes | Yes | 1 min |
| 13 | 40 F | 5 mins | 2 | Yes | Yes | 1 min |
| 14 | 43 F | 5 mins | 2 | Yes | Yes | 1 min |
| 15 | 61 F | 3 mins | 6 | Yes | Yes | 4 mins |
| 16 | 32 F | 4 mins | 4 | Yes | Yes | 90 secs |
| 17 | 38 F | 5 mins | 3 | Yes | Yes | 90 secs |
| 18 | 25 F | 5 mins | 2 | Yes | Yes | within 30 secs |
| 19 | 50 F | 5 mins | 4+2 | Yes | Yes | 1 minute, recurred after 10 mins, then resolved in 1 min |

**Table 7. Clinical Study Symptoms**

| **Patient** | **Laryn-geal edema / Throat Tightening** | **Hypotension** | **Headache** | **Light-headed / Dizzy** | **Urticaria** | **Tongue / Lip** | **Itchy Eyes** | **Itchy Ears** | **Facial Swelling / Rash** | **Chest Tightness/ Heaviness** |
|---|---|---|---|---|---|---|---|---|---|---|
| 1 | | | | | Yes | Tongue / Lip Tingling | | | Yes | |
| 2 | Yes | | | | Yes | Lip Tingling & Swelling | | | | |
| 3 | Yes | | | | | | Yes | | Yes | |
| 4 | Yes | | Yes | | | | Yes | | | |
| 5 | Yes | | Yes | Yes | | | | | | Yes |
| 6 | Yes | | | Yes | | | | Yes | | Yes |
| 7 | | | | Yes | Yes | | | | | |
| 8 | Yes | | | | | | | | | |
| 9 | Yes | | | | Yes | | | | | |
| 10 | Yes | | | Fuzzy Think-ing | Yes | | | | | Yes |
| 11 | Yes | | | Yes | | | | | | Yes |
| 12 | | | | Yes | Yes | | | | | |
| 13 | Yes | | | Yes | Yes | | | | | |
| 14 | Yes | | | | | | | | | Yes |
| 15 | Yes | | | Yes | | | | | | Yes |
| 16 | Yes | Yes | | Yes | | | | | | Yes |
| 17 | Yes | | | Yes | Yes | | | | | |
| 18 | Yes | | | Yes | Yes | | | | | Yes |
| 19 | Yes | | | Yes | | | | Yes | | |

Oral inhaled epinephrine provided prompt resolution of clinical symptoms, including both laryngopharyngeal and systemic symptoms, in all study participants. In two instances symptoms recurred after initial resolution and again resolved promptly after further doses of oral inhaled epinephrine were administered. No study participants required treatment with intramuscular epinephrine.

All of the U.S. patents, U.S. patent application publications, U.S. patent applications, foreign patents, foreign patent applications, and non-patent publications referred to in this specification, including U.S. Provisional Appl. No. 63/165,102, filed March 23, 2021.

Although the foregoing devices, compositions, and methods have been described in some detail to facilitate understanding, it will be apparent that certain changes and modifications may be practiced within the scope of the appended claims. Accordingly, the described embodiments are to be considered as illustrative and not restrictive, and the claimed invention is not to be limited to the details given herein, but may be modified within the scope and equivalents of the appended claims.

## Claims

1. A formulation for use in a method of treating an anaphylactic reaction, said formulation comprising:
an active pharmaceutical ingredient (API) selected from epinephrine and a pharmaceutically acceptable salt thereof;
a liquefied propellant; and
a co-solvent;
wherein the method comprises orally administering a therapeutically effective amount of epinephrine or a pharmaceutically acceptable salt thereof by administering a dose using a metered dose inhaler once every 1 to 10 minutes until the anaphylactic reaction ameliorates.

2. The formulation for use according to claim 1, wherein the method comprises administering two puffs once every 1 to 10 minutes.

3. The formulation for use according to claim 1 or claim 2, wherein the method comprises orally administering epinephrine or a pharmaceutically acceptable salt thereof once every 2 minutes.

4. The formulation for use according to claim 1, wherein two puffs are administered every 60 seconds until clinical resolution of allergic symptoms is obtained.

5. The formulation for use according to any one of claims 1-4, wherein two puffs are administered periodically until the patient shows a physiological response to the treatment.

6. The formulation for use according to claim 5, wherein the physiological response is:
(a) tachycardia, tremor or both; or
(b) the patient's subjective awareness of body or hand tremor or patient's subjective awareness of increased heart rate.

7. The formulation for use according to claim 1 or 2, wherein the method comprises administering by oral inhalation 250 µg epinephrine or a pharmaceutically acceptable salt thereof (for example two puffs) periodically (for example every 30-90 seconds, preferably every 45-75 seconds, more preferably about every 60 seconds) until the patient experiences subjective awareness of a physiological response to treatment (for example awareness of increased heart rate, awareness of body or hand tremor, or awareness of both increased heart rate and body or hand tremor).

8. The formulation for use according to any one of the preceding claims, wherein epinephrine or a pharmaceutically acceptable salt thereof is administered shortly after the onset of an anaphylactic reaction, optionally within 5 minutes, 4 minutes, 3 minutes, 2 minutes, or 1 minute after the onset of the reaction.

9. The formulation for use according to claim 8, wherein epinephrine or a pharmaceutically acceptable salt thereof is administered again if the anaphylactic reaction returns.

10. The formulation for use according to any one of the preceding claims, wherein the metered dose inhaler is a pressurized metered dose inhaler.

11. The formulation for use according to any one of the preceding claims, wherein the API is a suspension of API.

## Patentansprüche

1. Formulierung für die Verwendung bei einem Verfahren zum Behandeln einer anaphylaktischen Reaktion, wobei die Formulierung umfasst:
einen pharmazeutischen Wirkstoff (API), ausgewählt aus Epinephrin und einem pharmazeutisch unbedenklichen Salz davon;
ein verflüssigtes Treibmittel; und
ein Co-Lösungsmittel;
wobei das Verfahren das orale Verabreichen einer therapeutisch wirksamen Menge an Epinephrin oder einem pharmazeutisch unbedenklichen Salz davon durch Verabreichen einer Dosis unter Verwendung eines dosierten Dosisinhalators einmal alle 1 bis 10 Minuten, bis sich die anaphylaktische Reaktion bessert umfasst.

2. Formulierung für die Verwendung nach Anspruch 1, wobei das Verfahren das Verabreichen von zwei Sprühstößen einmal alle 1 bis 10 Minuten umfasst.

3. Formulierung für die Verwendung nach Anspruch 1 oder 2, wobei das Verfahren das orale Verabreichen von Epinephrin oder einem pharmazeutisch unbedenklichen Salz davon einmal alle 2 Minuten umfasst.

4. Formulierung für die Verwendung nach Anspruch 1, wobei alle 60 Sekunden zwei Sprühstöße verabreicht werden, bis eine klinische Besserung der allergischen Symptome erreicht ist.

5. Formulierung für die Verwendung nach einem der Ansprüche 1-4, wobei zwei Sprühstöße periodisch verabreicht werden, bis der Patient eine physiologische Reaktion auf die Behandlung zeigt.

6. Formulierung für die Verwendung nach Anspruch 5, wobei die physiologische Reaktion ist:
(a) Tachykardie, Tremor oder beides; oder
(b) das subjektive Empfinden des Patienten von Körper- oder Handtremor oder das subjektive Empfinden des Patienten einer erhöhten Herzfrequenz.

7. Formulierung für die Verwendung nach Anspruch 1 oder 2, wobei das Verfahren das Verabreichen durch orale Inhalation von 250 µg Epinephrin oder eines pharmazeutisch unbedenklichen Salzes davon (beispielsweise zwei Sprühstöße) periodisch (beispielsweise alle 30-90 Sekunden, vorzugsweise alle 45-75 Sekunden, stärker bevorzugt etwa alle 60 Sekunden) umfasst, bis der Patient eine subjektive Empfindung einer physiologischen Reaktion auf die Behandlung verspürt (beispielsweise die Empfindung einer erhöhten Herzfrequenz, die Empfindung eines Körper- oder Handtremors oder die Empfindung sowohl einer erhöhten Herzfrequenz als auch eines Körper- oder Handtremors).

8. Formulierung für die Verwendung nach einem der vorstehenden Ansprüche, wobei Epinephrin oder ein pharmazeutisch unbedenkliches Salz davon kurz nach dem Einsetzen einer anaphylaktischen Reaktion verabreicht wird, optional innerhalb von 5 Minuten, 4 Minuten, 3 Minuten, 2 Minuten oder 1 Minute nach dem Einsetzen der Reaktion.

9. Formulierung für die Verwendung nach Anspruch 8, wobei Epinephrin oder ein pharmazeutisch unbedenkliches Salz davon erneut verabreicht wird, falls die anaphylaktische Reaktion erneut auftritt.

10. Formulierung für die Verwendung nach einem der vorstehenden Ansprüche, wobei der dosierte Dosisinhalator ein unter Druck stehender dosierter Dosisinhalator ist.

11. Formulierung für die Verwendung nach einem der vorstehenden Ansprüche, wobei der API eine Suspension von API ist.

## Revendications

1. Formulation destinée à être utilisée dans un procédé de traitement d'une réaction anaphylactique, ladite formulation comprenant :
un principe pharmaceutique actif (API) choisi parmi l'épinéphrine et un sel pharmaceutiquement acceptable de celle-ci ;
un propulseur liquéfié ; et
un co-solvant ;
dans laquelle le procédé comprend l'administration par voie orale d'une quantité thérapeutiquement efficace d'épinéphrine ou d'un sel pharmaceutiquement acceptable de celle-ci, en administrant une dose au moyen d'un aérosol-doseur une fois toutes les 1 à 10 minutes jusqu'à ce que la réaction anaphylactique s'atténue.

2. Formulation destinée à être utilisée selon la revendication 1, dans laquelle le procédé comprend l'administration de deux bouffées une fois toutes les 1 à 10 minutes.

3. Formulation destinée à être utilisée selon la revendication 1 ou la revendication 2, dans laquelle le procédé comprend l'administration par voie orale d'épinéphrine ou d'un sel pharmaceutiquement acceptable de celle-ci une fois toutes les 2 minutes.

4. Formulation destinée à être utilisée selon la revendication 1, dans laquelle deux bouffées sont administrées toutes les 60 secondes jusqu'à ce que la résolution clinique des symptômes allergiques soit obtenue.

5. Formulation destinée à être utilisée selon l'une quelconque des revendications 1 à 4, dans laquelle deux bouffées sont administrées périodiquement jusqu'à ce que le patient présente une réponse physiologique au traitement.

6. Formulation destinée à être utilisée selon la revendication 5, dans laquelle la réponse physiologique est :
(a) une tachycardie, des tremblements ou les deux ; ou
(b) la perception subjective par le patient de tremblements du corps ou des mains ou la perception subjective par le patient d'une accélération du rythme cardiaque.

7. Formulation destinée à être utilisée selon la revendication 1 ou 2, dans laquelle le procédé comprend l'administration par inhalation orale de 250 µg d'épinéphrine ou d'un sel pharmaceutiquement acceptable de celle-ci (par exemple deux bouffées) périodiquement (par exemple toutes les 30 à 90 secondes, de préférence toutes les 45 à 75 secondes, plus préférablement environ toutes les 60 secondes) jusqu'à ce que le patient perçoive subjectivement une réponse physiologique au traitement (par exemple, perception d'une accélération du rythme cardiaque, perception de tremblements du corps ou des mains, ou perception à la fois d'une accélération du rythme cardiaque et de tremblements du corps ou des mains).

8. Formulation destinée à être utilisée selon l'une quelconque des revendications précédentes, dans laquelle l'épinéphrine ou un sel pharmaceutiquement acceptable de celle-ci est administré peu après le début d'une réaction anaphylactique, éventuellement dans les 5 minutes, 4 minutes, 3 minutes, 2 minutes, ou 1 minute après le début de la réaction.

9. Formulation destinée à être utilisée selon la revendication 8, dans laquelle l'épinéphrine ou un sel pharmaceutiquement acceptable de celle-ci est administré à nouveau si la réaction anaphylactique réapparaît.

10. Formulation destinée à être utilisée selon l'une quelconque des revendications précédentes, dans laquelle l'aérosol-doseur est un aérosol-doseur sous pression.

11. Formulation destinée à être utilisée selon l'une quelconque des revendications précédentes, dans laquelle l'API est une suspension d'API.
